# EUROPEAN PATENT APPLICATION

(11) **EP 1 862 467 A2**
(43) Date of publication of application: **05.12.2007**
(21) Application number: 06743410.0
(22) Date of filing: 23.03.2006
(51) Int. Cl.: C07H 17/08, C12P 19/62, A61P 31/00, C12R 1/465

(54) **POLYENE ANTIBIOTICS, COMPOSITIONS CONTAINING SAID ANTIBIOTICS, METHOD AND MICRO-ORGANISMS USED TO OBTAIN SAME AND APPLICATIONS THEREOF**

(30) Priority: 23.03.2005 ES 200500701; 03.08.2005 ES 200501952
(71) Applicant: Consejo Superior de Investigaciones Cientificas (CSIC), 28006 Madrid (ES)
(72) Inventor: MALPARTIDA ROMERO, Francisco,Cnto Nac. de Biotec., 28049 Madrid (ES); SECO MARTÍN, Elena M.,Cnto Nac. de Biotec., 28049 Madrid (ES); CUESTA VELASCO, Trinidad,Cnto Nac. de Biotec., 28049 Madrid (ES)
(74) Representative: Ungria Lopez, Javier
(86) International application number: PCT/ES2006/000142
(87) International publication number: WO 2006/100330

(57) **Abstract**

Abstract: The invention relates to novel polyenes having formula (I), wherein: R1 represents alkyl C₁-C₃; and R2 represents a functional group selected from CH₃- or CONH₂- (methyl- or primary amide-). The aforementioned polyenes have a biocide action on organisms comprising cell membranes that contain ergosterol, e.g., fungi or parasites. Said compounds can be obtained using a method that consists in cultivating a producing micro-organism under conditions that enable the production thereof. In addition, the invention also relates to a mechanism for the *in vitro* production of amidated polyenes, consisting in incubating carboxylated polyenes with cell-free extracts (or proteinaceous fractions) of the producers of same in the presence of ATP/Mg⁺⁺ and an amide-group donor compound (preferably glutamine).

## Description

### FIELD OF THE INVENTION

The invention relates to novel amidated and methylated polyenes, method for obtaining same, characterisation of biological activities and applications, for example, therapeutic, agricultural and agro-alimentary. The invention also relates to: (a) methylated derivatives of other polyenes obtained in the same way in their respective producing organisms; (b) recombinant producing micro-organisms of amidated polyenes as well as (c) vectors useful for obtaining said micro-organisms and (d) a method for obtaining amidated polyenes using cell-free extracts or proteinaceous fractions obtained from producers of amidated polyene macrolides.

### PRIOR ART OF THE INVENTION

In the past, fungal infections used to occupy a fairly unimportant place in the panorama of infectious diseases. Nevertheless, that panorama has altered radically in the last twenty years. The increase in immuno-depressed patients, bone marrow transplants and transplants of solid organs, the increased number of patients with cancer, chemotherapy treatments, the use of immunosuppressor agents and broad-spectrum antimicrobial agents along with other drugs that alter the natural defence mechanisms, and the AIDS epidemic, have all been responsible for this change. Nosocomial infection due to fungal species is becoming ever more important and the fungal species that are associated with deep-seated mycosis are also becoming more numerous.

In spite of the need to have antifungal drugs, the number of these pharmaceutical products on the market for treating systemic infections is dangerously low. The majority of them, such as azoles and polyenes, included among which is amphotericin B, are targeted at the structural integrity of the fungal membranes, though in recent years antifungal drugs (echinocandins) have been developed which are targeted at specific components of the cell wall.

Polyenes are a group of polyketone macrolides that are very interesting on account of their antifungal activity. These compounds contain a macrolactone ring with numerous conjugated double bonds forming chromophores with a characteristic spectrum in the ultraviolet/visible region; these characteristics are responsible for their physical and chemical properties (high absorption of light, photolability and low level of solubility in water). In spite of the importance of some of them such as amphotericin B as antifungal drugs, their precise mechanism of action is not yet well understood; nevertheless, the antifungal activity seems to be due to interactions between the polyene molecules and the membranes containing sterol. This interaction provides a channel of ions and the membranes become permeable causing destruction of the electrochemical gradients and consequent cell death. These compounds display a significantly greater affinity towards membranes containing ergosterol (the main sterol present in the membranes of fungi and parasites such as *Trypanosoma* and *Leishmania*) than membranes containing cholesterol (cells of mammals). Nevertheless, the interaction between the polyenes and membranes containing cholesterol is not insignificant and causes side-effects, which, together with the low solubility, means that the compound is not entirely satisfactory for treating systemic fungal infections. In spite of these undesirable properties and the toxic side-effects of amphotericin B, this old drug has been used for more than 40 years and continues to be the preferred antifungal agent for treating most systemic infections; in fact, it is accepted that there are no better alternatives available for fighting the emerging fungal infections. Some of the undesirable effects can be minimised by means of releasing the drug in a liposomal formulation; this reduces the toxicity of amphotericin B and has allowed its systemic application as an antifungal (mycosis) and antiparasitic (e.g., against leichmaniasis and trypanosimiasis, among other parasites), organisms whose cell walls contain ergosterol (Berman et al., 1992, Antimicrob. Agents Chemother 36: 1978-1980; Herwaldt (1999), The Lancet. 354: 1191-1199; Yardley et al., 1999, Am. J. Trop. Med. Hyg. 61: 163-197).

For this reason, the discovery of novel antifungal drugs or the improvement of the pharmacological properties of those already existing constitutes an exciting challenge. With this objective, and using rational approximations of molecular models, several semi-synthetic derivatives of amphotericin B have been generated and tested as efficient antifungal drugs. In order to carry out the structural modifications, two main targets have been considered, among others: the carboxyl- group of the side chain and the amino group in the sugar. Although some of these semi-synthetic derivatives still displayed the same toxicity, others presented improved pharmacological characteristics compared to the molecule of amphotericin B: greater antifungal activity, better solubility in water, greater specificity for membranes containing ergosterol and lower haemolytic activity, which suggested a greater specificity for membranes containing ergosterol. Although the greater antifungal activity confers some advantage on these compounds, surprisingly, these structural modifications are not commonly represented within natural polyenes isolated from micro-organisms. In fact, all the polyenes described so far as improved drugs are semi-synthetic derivatives, generated by organic synthesis rather than by biotransformations.

Although various non-polyene antifungal agents are available, the use of these drugs favours the selection and development of strains resistant to them which can in future compromise the efficiency of these compounds for antifungal treatment. So there exists widespread interest in the search for novel antifungal drugs and in improving the pharmacological properties of already existing ones.

### SUMMARY OF THE INVENTION

The inventors of the invention have found that by means of the genetic manipulation of *Streptomyces diastaticus* var. 108, a producing strain of polyene macrolide antibiotics rimocidin (IIa) and CE-108 (IIb) (Perez-Zuniga et al. (2004), J. Antibiot. (Tokyo) 57: 197-204), as well as the two native polyenes, novel polyene macrolides are also obtained in the fermentation culture of the recombinant strain which, after being characterised chemically, turned out to be the amides of the carboxylic acids, rimocidin B (I-1a) and CE-108B (I-1b) of the polyenes that they came from, rimocidin (IIa) and CE-108 (IIb), respectively, and methyls of formula (III), rimocidin C (IIIa) and CE-108C (IIIb), all of them included in formula (I). The biological activity and some toxicity tests *in vitro* showed that the chemical modifications present in the novel compounds confer improved biological properties compared to those shown by the products that they came from (see Example I and Example 2).

More specifically, the inventors of the invention have found that by means of the genetic manipulation of *Streptomyces diastaticus* var. 108, a producing strain of polyene macrolide antibiotics rimocidin (IIa) and CE-108 (IIb) (Perez-Zuniga et al. (2004), J. Antibiot. (Tokyo) 57: 197-204), and preferably by transformation with vectors derived from SCP2* which carry the erythromycin resistance gene (*ermE*) (Uchiyama et al., (1985), Gene 38: 103-110) as well as the two native polyenes, novel polyene macrolides are also obtained in the fermentation culture of the recombinant strain which, after being characterised chemically, turned out to be the amides of the carboxylic acids, rimocidin B (I-1 a) and CE-108B (I-1b) of the polyenes that they came from, rimocidin (IIa) and CE-108 (IIb), respectively. The biological activity and some toxicity tests *in vitro* showed that the chemical modifications present in the novel compounds (amide in place of carboxylic acid) confer improved biological properties compared to those shown by the products that they came from (Table 2, Example I).

In this invention, moreover, the biosynthetic mechanism is elucidated for the formation of these amidated polyenes rimocidin B (I-1a) and CE-108B (I-1 b), mentioned above, and a description is given of the method of obtaining two novel polyenes rimocidin C (IIIa) and CE-108C (IIIb) (methylated polyenes, Example 2) by means of the genetic manipulation of the cluster involved in the biosynthesis of the two polyene macrolide antibiotics rimocidin (IIa) and CE-108 (IIb) (Seco et al., 2004, Chem. Biol. 11: 357-366). The novel methylated polyenes, as with the amidated ones, display clearly improved pharmacological properties with respect to the native tetraenes.

On the basis of the model proposed for the biosynthesis of the native tetraenes rimocidin (IIa) and CE-108 (IIb) (Seco et al., 2004, Chem. Biol. 11: 357-366), module 7 of the polyketide synthetase (PKS) incorporates methylmalonamyl-CoA as elongation unit, which is responsible for the presence of a methyl- group in the macrocyclic ring which, later on, by means of a site-specific post-PKS modification, would become oxidised to give the free carboxyl- group present in rimocidin (IIa) and CE-108 (IIb). It is described that a cytochrome P450 monooxygenase is involved in this oxidation and is coded in the biosynthetic clusters of polyenes described so far (Aparicio et al., 2003, Appl Microbiol Biotechnol 61: 179-188). In the case of the biosynthesis of rimocidin (IIa) and CE-108 (IIb), owing to the similarity of its sequence and to the fact that just one single cytochrome P450 monooxygenase is required in the biosynthetic model, it was proposed that RimG was the cytochrome P450 monooxygenase involved in the oxidation of the methyl- group in order to originate the carboxyl- group (Seco et al., 2004, Chem. Biol. 11: 357-366).

For the formation of the amide- group present in the amidated polyenes rimocidin B (I-1a) and CE-108B (I-1b), two possible mechanisms were initially proposed as a theory: (**a**) incorporation of malonamyl-CoA units during the assembly of the polyketone chain in place of methylmalonyl-CoA units proposed for the formation of rimocidin (IIa) and CE-108 (IIb) or (**b**) amidotransferase activity acting once the side methyl- group of the macrolactane ring is oxidised to the carboxyl- group.

The invention relates to two amidated polyenes identified later on in this description as rimocidin B (I-1a) and CE-108B (I-1b) and two methylated polyenes identified later on as rimocidin C (IIIa) and CE-108C (IIIb); their method of obtaining and applications constitute additional aspects of this invention.

Said compounds have biocide activity which is in general more selective against organisms that have cell membranes containing ergosterol, either fungi or parasites. Biocide compositions, for example pharmaceutical compositions and/or antifungal compositions for agricultural or agro-alimentary use containing said amidated or methylated polyenes, constitute an additional aspect of this invention. The use of such polyenes, whether they be amidated or methylated, in pharmaceutical compositions with sanitary ends for human or animal use and/or antifungal compositions for agricultural or agro-alimentary use constitute another aspect of this invention.

In another aspect, the invention relates to applications of the polyene AB-400 (IVb), the corresponding amide of pimaricin (IVa) (Cañedo L. M. et al. 2000, J., Antibiot. (Tokyo) 53: 623-626), based on the results of tests of this compound, isolated from *Streptomyces* sp. RGU5.3 and highlighted in this invention.

In another aspect, the invention relates to the use of vectors derived from SCP2* containing at least the erythromycin resistance gene (*ermE*)*,* such as pSM784 or pSM743B, identified further below. The use of said vectors in order to start from producing micro-organisms of polyene macrolides containing a free carboxyl- and generate recombinant producing micro-organisms of polyene macrolides containing an amide- group in place of the carboxyl- group constitutes another aspect of this invention.

The invention relates in another aspect to a method for the production of said amidated polyenes consisting of cultivating a recombinant producing micro-organism of said compounds under conditions that permit the production of said polyenes, be they amidated or methylated, and, if desired, to isolate and purify those compounds. Illustrative examples of such recombinant micro-organisms include *Streptomyces diastaticus* var. 108/784, *Streptomyces diastaticus* var. 108/743B and *Streptomyces diastaticus* var. 108::PM1-500/743B (see examples), which constitute an additional aspect of this invention along with the use of them or similar recombinant micro-organisms in obtaining said amidated polyenes and, optionally, in obtaining mixtures of these with polyenes containing a carboxyl- group.

In another aspect, the invention relates to the elucidation of the mechanism for the formation of the amide- group of the amidated polyenes, for which an interruption or deletion of the gene *rimG* with the aim of blocking the formation of the carboxyl- group was crucial. Once the formation of the carboxyl- group had been blocked, if the formation of the amide- group takes place during the assembly of the polyketone chain then, when transforming the disrupting strain with an inducer plasmid of the biosynthesis of the amidated tetraenes, the latter ought to be detected in the fermentation culture. In the negative case, the conclusion would be that the formation of the amide- group takes place by means of an amidotransferase activity on the free carboxyl-group once the latter has been formed.

Inactivating gene disruption was chosen as the alternative for mutagenising the gene *rimG.* Nevertheless, in this initial conditions, and unexpectedly, the disruption in the gene *rimG* carried out as stated above generates a recombinant incapable of producing polyenes. This was interpreted to mean that the promoter used for the disruption was incapable of preventing polar effects probably on the gene *rim*A located after the insertion point. After that, an interruption in this gene rimG in the chromosome in a strain where the plasmid pSM743B was present (capable of complementing a polar effect in the gene *rimA* located downstream in the chromosome, in addition to inducing the formation of the amidated tetraenes) permitted the isolation of two novel methylated polyenes which have been given the names rimocidin C (IIIa) and CE-108C (IIIb), which display a substitution of the free carboxyl- group for a methyl- group as a consequence of the interruption of the gene *rimG.* The absence of amidated tetraenes in the fermentation culture of the resulting strain (*Streptomyces diastaticus* var. 108::PM1-768/743B) (deposit No. DSM 17482) permitted the conclusion to be drawn that the formation of the amide- group in rimocidin B (I-1a) and CE-108B (I-1b) takes place by means of a site-specific post-PKS modification once the macrocyclic ring and the free side carboxyl-group have been formed, and not during the elongation of the polyketone chain, and that it is due to an "adornment" activity due probably to an amidotransferase.

Moreover, tests of biological activity towards various strains of fungi *(Penicillium chrysogenum, Candida krusei, Aspergillus niger, Candida albicans, Cryptococcus neoformans)* and some toxicity trials *in vitro* show that the chemical modification present in the novel compounds confers a clear pharmacological advantage with respect to the native polyenes that they came from (Table 2, Example 2).

Therefore, the invention also relates to a methylated polyene of formula (III), hereinafter the inventive compound, useful as an antifungal and antiparasite, and as particular objects of said polyene the following methylated polyenes are described identified further below in this specification as rimocidin C (IIIa) and CE-108C (IIIb).

The invention relates in another aspect to a method for the production of said methylated polyenes of formula III which comprises cultivating the disrupting producing micro-organism of those compounds under conditions which permit the production of said methylated polyenes, and, if wished, to isolate and purify those compounds. In particular, it includes *Streptomyces diastaticus* var. 108::PM1-768/743B) (deposit No. DSM 17482, the inventive micro-organism), producer of rimocidin C (IIIa) and CE-108C (IIIb), which constitutes an additional aspect of this invention along with the use thereof or of similar disrupting micro-organisms in obtaining said methylated polyenes.

In another aspect, the invention also relates to the use of the interruption of genes involved in the same chemical modification as proposed for RimG (some of which have already been described) in other biosynthetic clusters of polyenes with the aim of producing the corresponding methylated derivatives.

The pharmacological advantage of the inventive methylated polyenes rimocidin C (IIIa) and CE-108C (IIIb) is based on a greater selective toxicity towards membranes containing ergosterol, either fungi or parasites, than towards human cell membranes, which notably reduces its toxicity. Biocide compositions, for example, pharmaceutical compositions and/or antifungal compositions for sanitary, agricultural or agro-alimentary use, containing those methylated polyenes, constitute an additional aspect of this invention.

Another additional aspect of the invention is the use of a biocide composition of the invention for treatment of infectious diseases in the field of human and animal health, agricultural and alimentary.

Moreover, by means of *in vitro* amidation trials with cell-free extracts of the producers of amidated polyenes (*S. diastaticus* var. 108/743B, *S. diastaticus* var. 108/784) and using rimocidin (IIa) and CE-108 (IIb) as substrates, it has been possible to conclude that the formation of the amide-group is due to an ATP/Mg⁺⁺ dependent amidotransferase activity capable of using glutamine as amide- group donor (see Example 2, section B). The same trials conducted with cell-free extracts of *Streptomyces* sp. RGU5.3 has permitted it to be confirmed that the polyene AB-400 (IVb), the amide of pimaricin (IVa), originates by means of an amidotransferase activity very similar to that of *S. diastaticus* var. 108, which acts on the free carboxyl- group of pimaricin in order to transform it into an amide- group in a reaction that is also ATP/Mg⁺⁺ dependent.

Moreover, if has furthermore been possible to conclude that the amidotransferase activity present in cell-free extracts of the genetic recombinants *S. diastaticus* var. 108 presents a relaxed specificity towards the substrate being capable of modifying not only the native substrates rimocidin (IIa) and CE-108 (IIb) in their corresponding amides rimocidin B (I-1a) and CE-108B (I-1b) but it is also capable of recognising heterologous substrates such as pimaricin (IVa). This did not occur for the amidotransferase activity of *Streptomyces* sp. RGU5.3, which was only capable of recognising pimaricin (IVa) as a substrate under the tested conditions.

The free carboxyl- group is fairly well preserved in the majority of typical polyenes, such as amphotericin B, nystatin, pimaricin, candicidin, etc. The substitution of these carboxyl- group for amide- groups would probably give rise to amidated polyenes with improved pharmacological properties. Therefore, in this aspect, the invention provides an enzymatic method, hereinafter the inventive enzymatic method, for converting carboxylated polyenes into the corresponding amidated ones, using cell-free extracts of the producers or compounds selected either directly or immobilised on suitable supports following the technology of the field of systems immobilised starting from a substrate and under certain culture conditions.

In another aspect, the invention relates to cell-free extracts of producers of amidated polyenes, carriers of an amidotransferase activity, capable of converting *"in vitro"* carboxylated polyenes into their corresponding amides necessary for commencing the inventive enzymatic method.

In another particular aspect, the invention relates to the cell-free extracts of the micro-organisms *S. diastaticus* var. 108/743B, *S. diastaticus* var. 108/784 - both of them producers of the amidated polyenes CE-108B (I-1b) and rimocidin B (I-1a) - carriers of an amidotransferase activity with the capacity for converting rimocidin (IIa) and CE-108 (IIb) as well as other heterologous substrates into their corresponding amides; and the cell-free extract of the micro-organisms *Streptomyces* sp. RGU5.3, the producer of the amidated polyene AB-400 (IVb), capable of converting at least pimaricin (IVa) into its corresponding amidated polyene AB-400 (IVb) under the tested conditions.

### BRIEF DESCRIPTION OF THE FIGURES

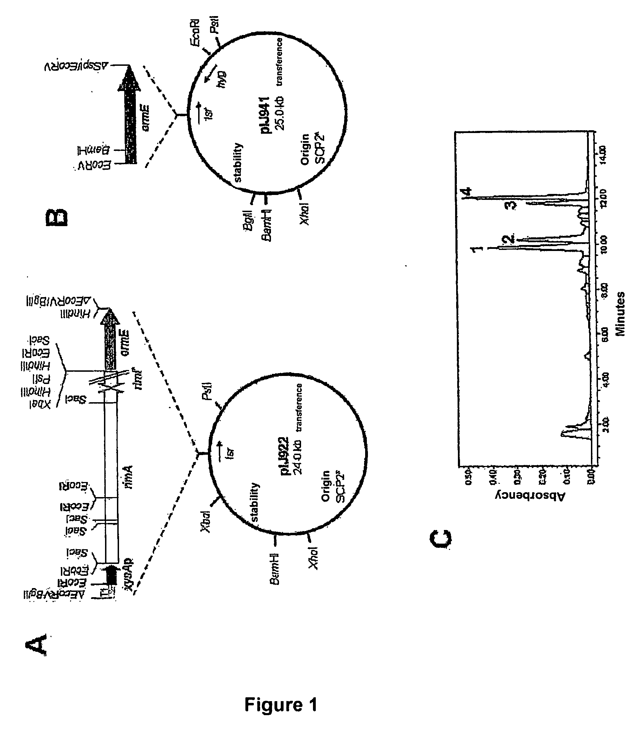

**Figure 1** shows a diagrammatic representation of the physical maps of the recombinant plasmids pSM743B (gene *rimA* introduced in pIJ922) [Figure 1 A] and pSM784 (gene *ermE* introduced in pIJ941) [Figure 1 B]. In those Figures 1 A and 1B, *xysAp:* promoter *zysA; rimA:* PKS Type I involved in the biosynthetic pathway of CE-108 and rimocidin; Ti: terminator of the methylenomycin resistance gene; *ermE, tsr* and *hyg:* resistance genes to erythromycin, thiostrepton and hygromycin respectively; *riml*: riml* truncated in its N-terminal. Figure 1C shows the chromatographic profile (HPLC) of the fermentation culture of the genetically modified strain *Streptomyces diastaticus* var. 108/743B. In said Figure 1C, the numbers 1-4 represent 1: CE-108B (I-1b); 2: CE-108 (IIb); 3: rimocidin B (I-1a) and 4: rimocidin (IIa).

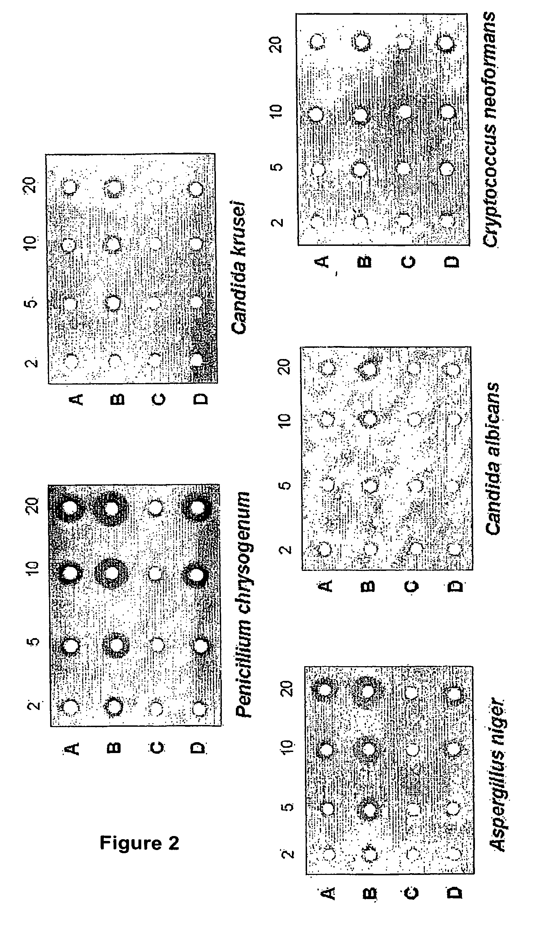

**Figure 2** shows the antifungal activity of four tetraenes produced by *S. diastaticus* var. 108/743B. In said figure, A: rimocidin (IIa), B: rimocidin B (I-1a), C: CE-108 (IIb), D: CE-108B (I-1b). The numbers appearing on each of the antibiograms refer to the quantities applied of each of the polyenes, expressed in nanomoles. The target organisms were: *P. chrysogenum, C. krusei, A. niger, C. albicans* and *C. neoformans* (see Table 1, Example 1).

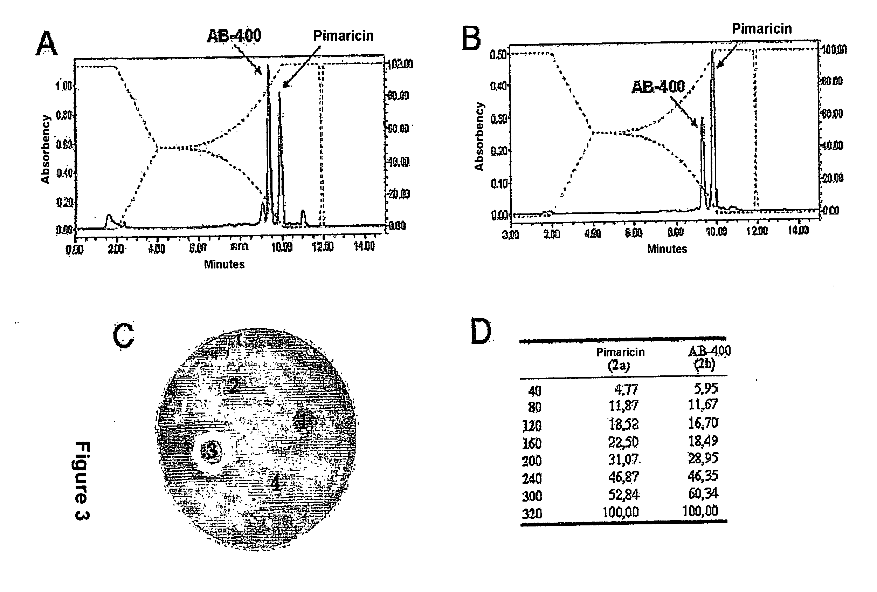

**Figure 3** shows the biological activities of the polyenes pimaricin (IVa) and AB-400 (IVb) isolated from *Streptomyces* sp. RGU5.3. It shows the HPLC analysis (chromatograms) of the fermentation cultures coming from *Streptomyces* sp. RGU5.3 grown in medium with acetate (Figure 3A) or glucose (Figure 3B) as source of carbon, respectively. Figure 3C illustrates the antifungal activity of pimaricin (IVa) and AB-400 (IVb) towards *P. chrysogenum;* the samples of polyenes applied were (1): commercial pimaricin (Calbiochem 5279962), (2): total extract of the fermentation culture of *S.* sp. RGU5.3 grown in glucose medium; (3) purified AB-400 coming from *S.* sp. RGU5.3 and (4) pimaricin coming from *S.* sp. RGU5.3; a total of 200 ng were added in each test. Figure 3D shows the haemolytic activity for pimaricin (Calbiochem 527962, purity 98.8%) and AB-400 (purified by HPLC as indicated in the Experimental Methods); the values of each polyene are expressed in nanomoles (left-hand column) and the corresponding haemolytic activities are provided as a percentage of total haemolysis (see the text for the experimental methods).

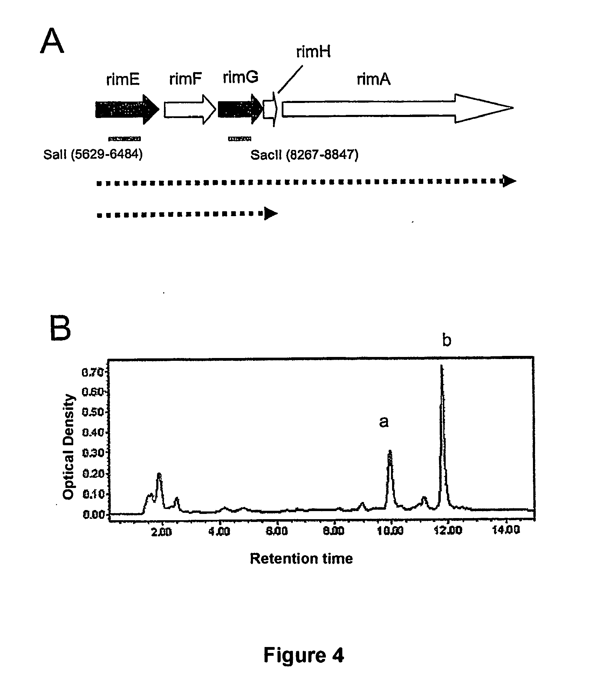

**Figure 4** illustrates, in panel A, a diagram of the gene transcription in this specific region of the chromosome deduced by means of protection assays on the endonuclease S1 conducted with different fragments of DNA (Seco et al., 2004, Chem. Biol. 11: 357-366). The transcripts are represented by broken arrows corresponding to the polycistron of the genes *rimE, rimF, rimG, rimH* and *rimA.* The transcripts deduced for the inactivating insertions of the genes *rimG* and *rimE* are indicated with a grey line; the numbers in brackets correspond to the DNA sequence deposited under access number AY442225. Panel B of this figure shows the chromatogram corresponding to the fermentation culture of *S. diastaticus* var. 108::PM1-768/743B, where a and b correspond to CE-108C (IIIb) and rimocidin C (IIIa) respectively.

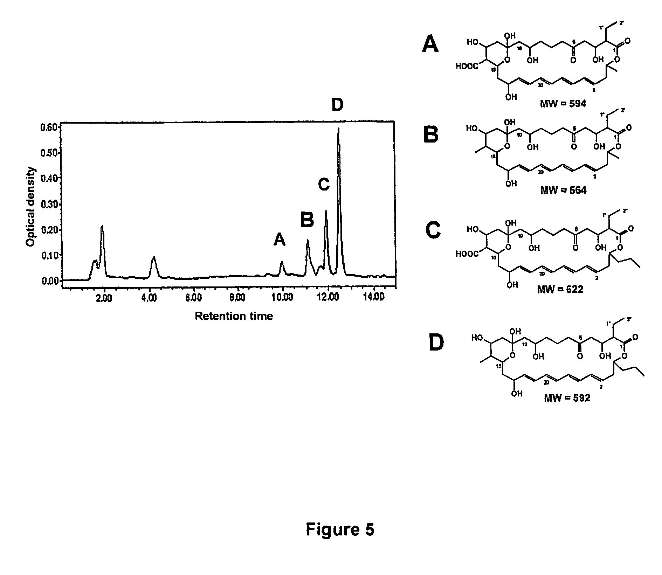

**Figure 5** represents, in the left-hand panel, the chromatogram of the fermentation culture of *S. diastaticus* var. 108::PM1-702B/743B. On the right of the chromatogram is the chemical structure deduced from the compounds detected in the chromatograph. The chemical structure was deduced on the basis of mass spectrometry analyses.

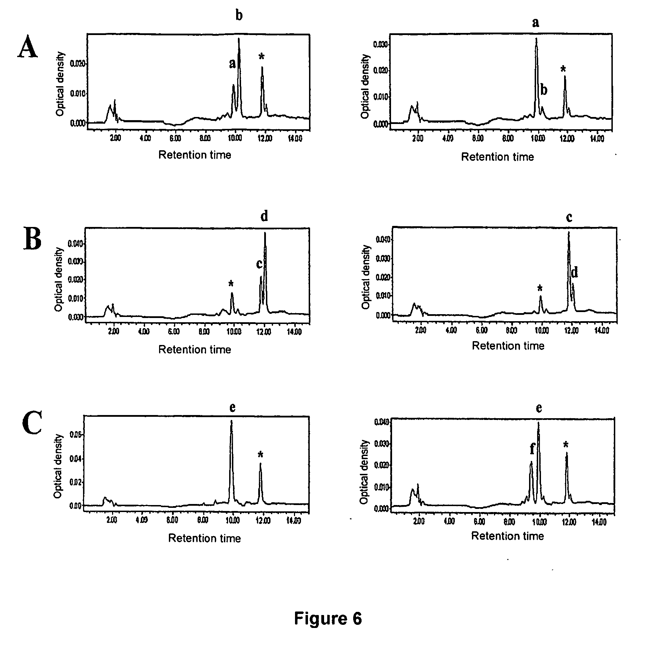

**Figure 6** shows the HPLC analysis of the *in vitro* amidotransferase trials conducted with cell-free extracts of *S. diastaticus* var. 108/784. The chromatograms of the left- and right-hand columns show the reaction to incubation times of 0 and 60 minutes, respectively. The peaks are: **a**. CE-108B; **b**. CE-108; **c**. rimocidin B; **d**. rimocidin; **e**. pimaricin; f. AB-400.
   A. Enzymatic conversion of CE-108 (IIb) into its amide CE-108B (I-1b). The peaks **a** and the one marked with an asterisk correspond to CE-108B (I-1b) and rimocidin B (I-1a) respectively, which it has not been possible to eliminate completely from the cell-free extract.
   B. Enzymatic conversion of rimocidin (IIa) into its amide rimocidin B (I-1a). The peaks **c** and the one marked with an asterisk correspond to rimocidin B (I-1a) and CE-108B (I-1b) respectively, present in the cell-free extract.
   C. Enzymatic conversion of pimaricin (IVa) into its amide AB-400 (IVb). The peaks marked with an asterisk correspond to rimocidin B (I-1a) present in the cell-free extract. Note the relaxed specificity to the substrate recognition by the amidotransferase present in the cell-free extract of *S. diastaticus* var. 108/784, being capable of converting a heterologous substrate (pimaricin) into its corresponding amide (AB-400).

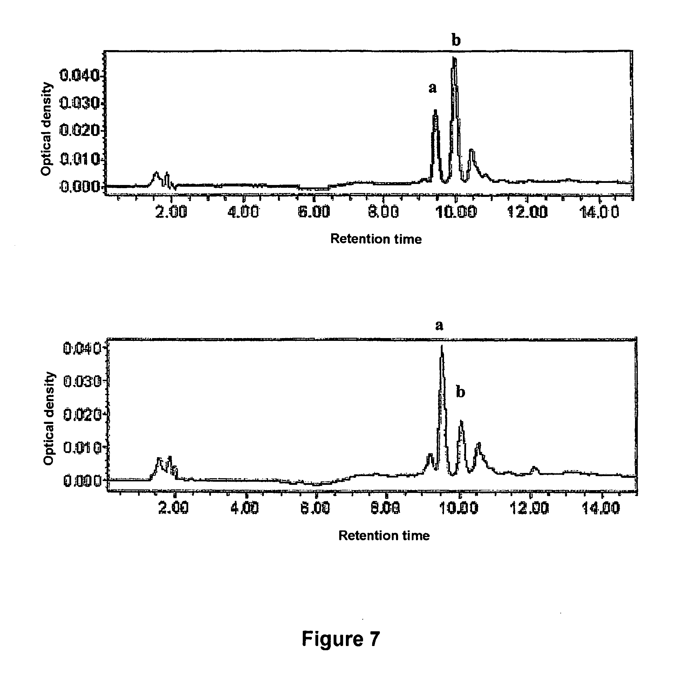

**Figure 7** shows the HPLC analyses of *in vitro* amidation trials on pimaricin (IVa) conducted with cell-free extracts of *S.* sp. RGU5.3. The chromatograms of the left- and right-hand columns show the reaction to incubation times of 0 and 60 minutes, respectively. The peak a present at time zero corresponds to AB-400 (IVb) present in the cell-free extract of *S.* sp. RGU5.3, which it has not been possible to eliminate completely. Note the clear conversion of pimaricin (IVa) (peak **a**) into its corresponding amide AB-400 (IVb) (peak **b**).

### DETAILED DESCRIPTION OF THE INVENTION

The present invention describes a novel polyene macrolide compound characterised by the formula (I): 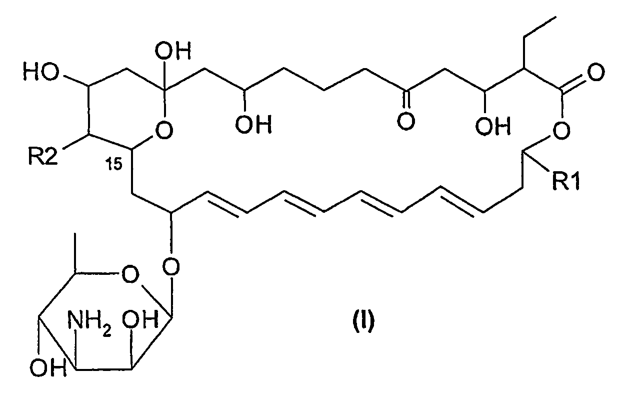 in which
R1 is alkyl C₁-C₃;
R2 is a functional group chosen between CH₃- or CONH₂- (methyl- or primary amide-), its isomers, salts, prodrugs or solvates.

The compounds of the present invention represented by formula (I) described above can include isomers, depending on the presence of multiple bonds (for example, Z, E), including optical or enantiomeric isomers, depending on the presence of chiral centres. The individual isomers, enantiomers or diastereoismers and mixtures of them fall within the scope of the present invention. The individual enantiomers or diastereoismers and mixtures of them can be separated by means of conventional techniques.

As used here, the term "salt" includes both pharmaceutically acceptable salts, in other words, salts of the compound of formula (I) that can be used in the preparation of a medicine, as well as pharmaceutically unacceptable salts, since these can be used in the preparation of pharmaceutically acceptable salts. The nature of the pharmaceutically acceptable salt is not critical always provided it is pharmaceutically acceptable. Among the pharmaceutically acceptable salts of compounds of formula (I) are to be found acid addition salts, which can be obtained starting from organic or inorganic acids, using conventional methods well known to technicians in the field by causing the appropriate acid to react with the compound of formula (I) in the stoichiometrically appropriate amount. Illustrative examples, though without being limiting, of acids that can be used to obtain said acid addition salts include organic acids, for example, acetic acid, ascorbic acid, citric acid, fumaric acid, maleic acid, methanesulphonic acid, oxalic acid, succinic acid, tartaric acid, p-toluenesulphonic acid, etc., or inorganic acids, for example, hydrobromic acid, hydrochloric acid, phosphoric acid, nitric acid, sulphuric acid, etc.

Likewise, within the scope of this invention are to be found the prodrugs of compounds of formula (I). The term "prodrug" as used here includes any compound derived from a compound of formula (I), for example, esters, including esters of carboxylic acids, esters of amino acids, phosphate esters, sulphonate esters of metallic salts, etc., carbamates, amides, etc., which, when administered to an individual, is capable of directly or indirectly delivering said compound of formula (I) in the said individual. Advantageously, said derivative is a compound that increases the bioavailability of the compound of formula (I) in a biological compartment. The nature of said derivative is not critical always provided it can be administered to an individual and delivers the compound of formula (I) in a biological compartment of an individual. The preparation of said prodrug can be carried out by means of conventional methods known to experts in the field.

The compounds of the invention can be in crystalline form as free compounds or as soivates and the aim is for both forms to come within the scope of this invention. In this regard, the term "solvate", as used here, includes both pharmaceutically acceptable solvates, in other words, solvates of the compound of formula (I) can be used in the preparation of a medicine, as well as pharmaceutically unacceptable solvates, since these can be used in the preparation of pharmaceutically acceptable solvates or salts. The nature of the pharmaceutically acceptable solvate is not critical always provided it is pharmaceutically acceptable. In a particular embodiment, the solvate is a hydrate. The solvates can be obtained by conventional methods well known to technicians in the field.

For their application in therapy, the compound of formula (I), their isomers, salts, prodrugs or solvates, will preferably come in a pharmaceutically acceptable or substantially pure form, in other words, which have a pharmaceutically acceptable level of purity excluding the normal pharmaceutical additives such as diluents and carriers, and not including material considered toxic at normal dosage levels. The levels of purity for the active principle is preferably greater than 50%, more preferably greater than 70%, more preferably greater than 90%. In a preferred embodiment, they are greater than 95% of the compound of formula (I), or of its salts, solvates or prodrugs.

Unless stated otherwise, the compounds of the invention also include compounds differing only in the presence of one or more isotopically enriched atoms. For example, compounds which have that structure, with the exception of the substitution of a hydrogen for a deuterium or tritium, or the substitution of a carbon for a carbon enriched in ¹³C or ¹⁴C or a nitrogen enriched in ¹⁵N, come within the scope of this invention.

In another aspect, the invention relates to a compound of formula (I-1), an amidated derivative of the polyene of the invention of formula (I): 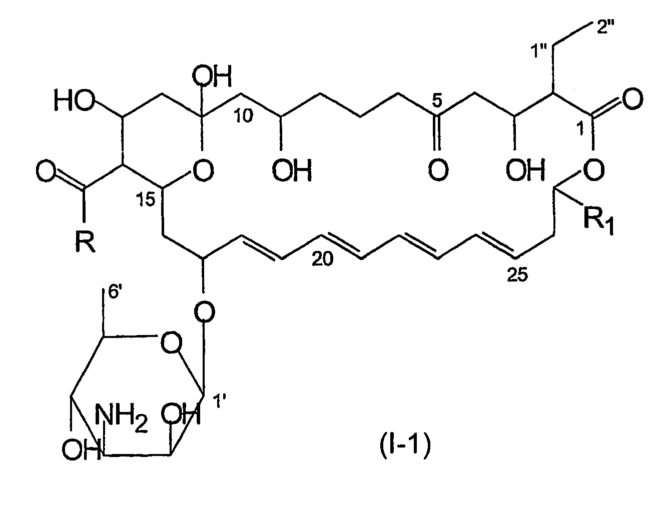 in which
R is NH₂; and
R1 is alkyl C₁-C₃,
or an isomer, salt, prodrug or solvate thereof.

In a particular embodiment, said compound of formula (I-1), is selected from the group formed by compounds identified in this description as "rimocidin B" of formula (I-1a) and "CE-108B" of formula (I-1b): 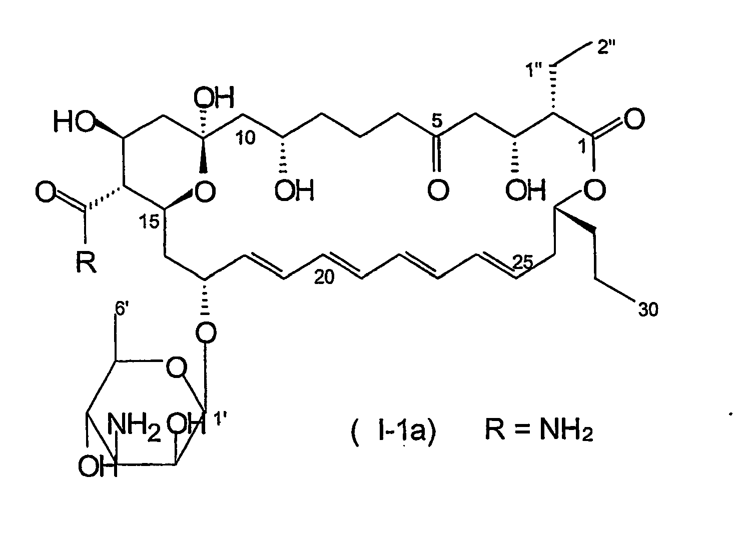 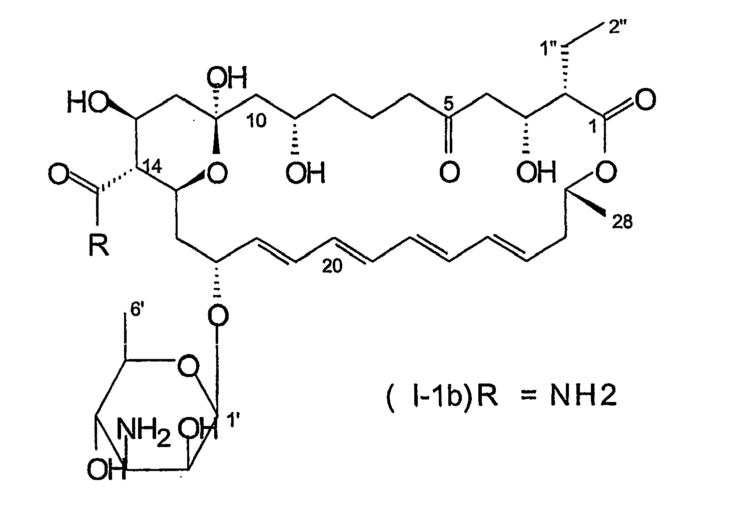 their isomers, salts, prodrugs or solvates.

These two novel amidated polyenes, rimocidin B (I-1a) and CE-108B (I-1 b), have been named thus because they are the corresponding amides of the natural compounds rimocidin (IIa) and CE-108 (IIb).

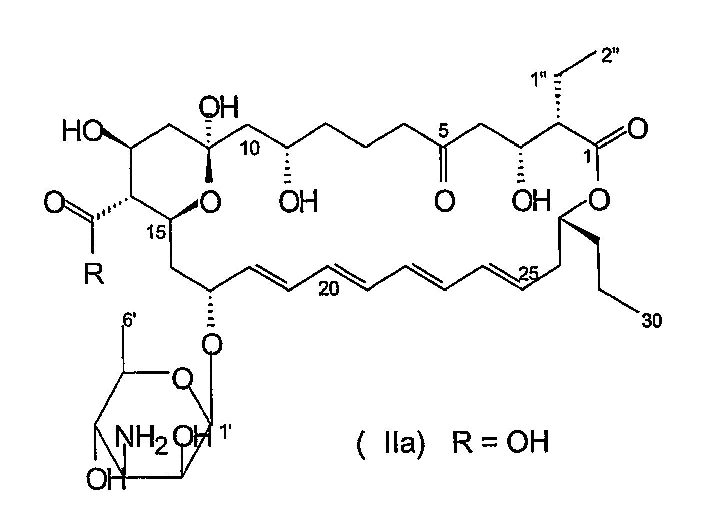

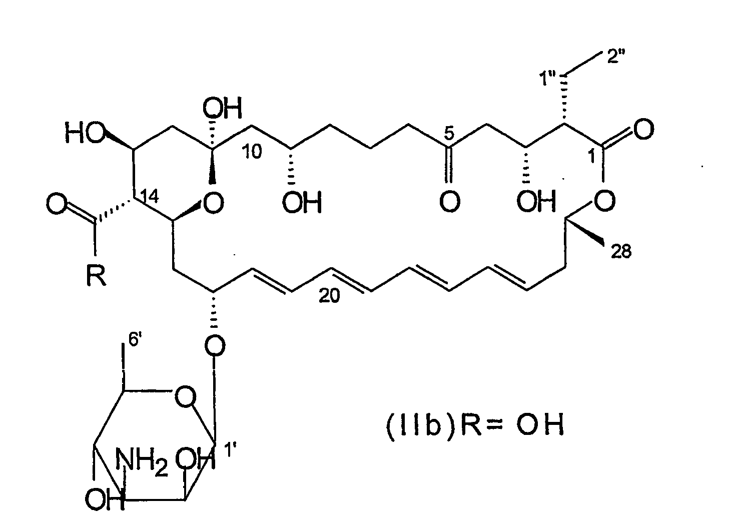

The chemical structure of these compounds has been characterised by means of various techniques as has their biological activity (for example, spectrometry, NMR, antibiograms, haemolytic activity, etc.). The substitution of the free carboxyl- group, present in the macrolactone ring of the rimicidin and CE-108, for an amide- group seems to lead to a greater affinity towards membranes with ergosterol (such as that of fungi and other organisms like the parasites *Trypanosoma* and *Leishmania*) than towards membranes with cholesterol (present in animal cells). This chemical modification increases the selective toxicity of these polyenes towards fungi, thus constituting a clear advantage with regard to their clinical application. In fact, the fungicide activity trials (Figure 2) and toxicity trials towards animal cells (erythrocytes) (Table 2, Example 1) reveal very similar toxicity values towards animal cells but highly significant increases in their biological activities, which leads to a greater selective toxicity towards fungi than the parent tetraenes rimicidin and CE-108. A similar specificity is applicable to other organisms with ergosterol in their membranes, such as the parasites *Leishmania* and *Trypanosoma.* Both amidated tetraenes also turned out to be more soluble than their corresponding parent compounds. Similar trials were also conducted with AB-400 (Cañedo L. M. et al. 2000, J., Antibiot. (Tokyo) 53: 623-626) (Figure 3), revealing an improvement in their pharmacological properties with respect to their non-amidated homologue (pimaricin) in addition to an increase in their solubility, and it can therefore be used as an antifungal/antiparasite medicine for treatment of systemic infections.

On another matter, the invention also relates to a compound of formula (III), a methylated derivative of the polyene of the invention of formula (I): 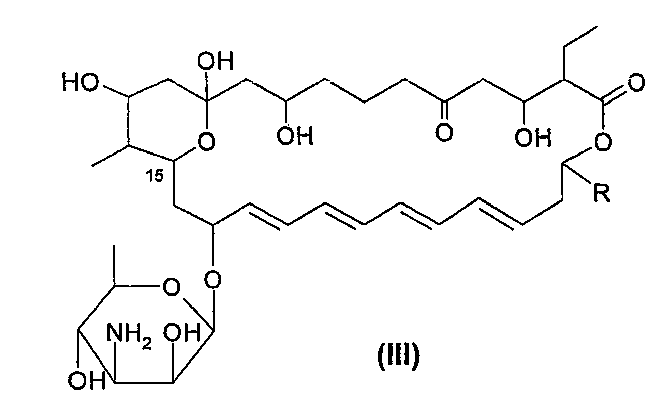 in which R is alkyl C₁-C₃, or an isomer, salt, prodrug or solvate thereof.

In a particular embodiment, said compound of formula (III) is selected from the group formed from compounds identified in this description as "rimocidin C" of formula (IIIa) and "CE-108" of formula (IIIb).

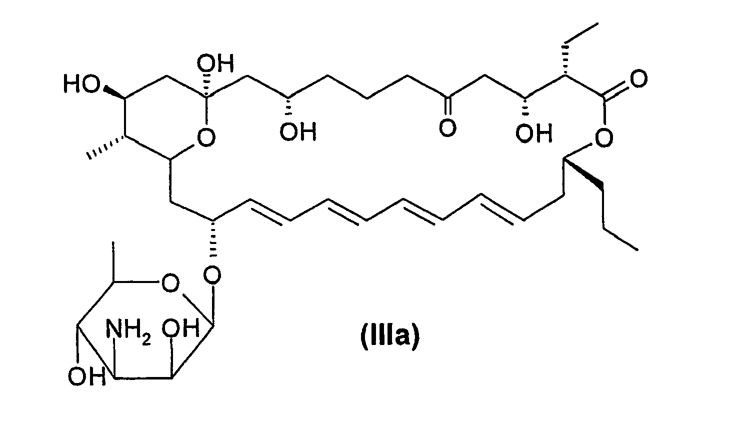

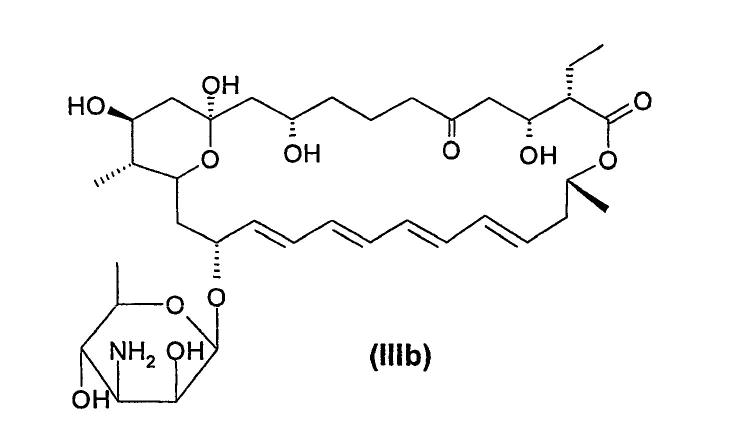

The two novel methylated polyenes, rimocidin C (IIIa) and CE-108C (IIIb), have been named thus because they are methylated polyenes derived from the natural compounds rimocidin (IIa) and CE-108 (IIb) in which the carboxyl- side group is substituted for a methyl-.

The chemical structure of these compounds has been characterised by means of various techniques as has their biological activity (for example, spectrometry, NMR, antibiograms, haemolytic activity, etc.). Unlike with amidated polyenes, the antifungal activity is not increased with respect to the parent compounds rimicidin and CE-108; nevertheless, the average toxicity in haemolytic terms is indeed lower than that of the compounds rimicidin and CE-108, which likewise implies an increase in the selective toxicity towards fungal membranes with respect to native polyenes (Example 2).

### Application of the novel amidated and methylated polyenes

The compounds of formula (I), and among them the compounds of formula (I-1) and (III), in general, have biocide activity and, in particular, biocide activity against organisms possessing a cell membrane containing ergosterol, and they are therefore potentially useful as biocides. As used in this description, a "biocide" is a chemical substance that halts the growth or kills different types of living beings.

Likewise, the expression "organisms possessing a cell membrane containing ergosterol" includes any organism endowed with a cell membrane containing ergosterol, for example, fungi, parasites, etc. Illustrative examples, though without being limiting, of such organisms include the parasites *Trypanosoma, Leishmania,* etc., along with fungi such as *Fusarium oxypsorium, Botrytis cinerea* (phytopathogens), *Candida albicans, Candida cruzei, Aspergillus niger, Cryptococcus neoformans* (human pathogens), among others.

Therefore, said compounds of formula (I), and among them the compounds of formula (I-1) and (III), and in particular the compounds rimocidin B (I-1a), CE-108B (I-1b), rimocidin C (IIIa) and CE-108C (IIIb), are potentially useful as biocides against said organisms possessing a cell membrane containing ergosterol. In a particular embodiment, said compounds are more useful as antiparasite agents or as antifungal agents than the corresponding non-amidated polyenes. The term "antifungal" includes both fungicides and fungistatics.

As a consequence, in another aspect, the invention relates to a biocide composition comprising a compound of formula (I), and among it the compounds of formula (1-1) and (III), together with an inert vehicle. In a particular embodiment, said compound of formula (I) is selected from the group of formula (I-1) and preferably rimocidin B (I-1a), CE-108B (I-1b) and their mixtures; in another embodiment, said compound of formula (III) is selected from the group of formula (III) and preferably rimocidin C (IIIa), CE-108C (IIIb) and their mixtures. Said biocide composition is particularly useful against organisms possessing a cell membrane containing ergosterol. In a particular embodiment, said biocide composition is an antifungal composition comprising a compound of formula (I) such as a compound selected from among the compounds rimocidin B (I-1a), CE-108B (I-1b), rimocidin C (IIIa) and CE-108C (IIIb) and their mixtures, optionally together with one or more inert vehicles.

As used in this description, the term "inert" means that said vehicle has no significant biocide activity.

If wished, said composition can furthermore contain other natural, recombinant or synthetic biocides, which might possibly strengthen the biocide action of said compound of formula (I) for example, the compounds rimocidin B (I-1a) and/or CE-108B (I-1b), rimocidin C (IIIa), CE-108C (IIIb) and their mixtures, or increase the spectrum of action.

### 1.- Therapeutic applications

An important field where the compounds of formula (I), and in particular the compounds rimocidin B, CE-108B, rimocidin C and CE-108C, find application is in human and animal health. Therefore, in a particular embodiment, the invention provides a pharmaceutical composition that comprises a compound of formula (I), and among them a compound of formula (I-1) or (III) or their mixtures, optionally together with one or more pharmaceutically acceptable excipients. In a particular embodiment, said compound of formula (I) is selected from the compounds rimocidin B (I-1a), CE-108B (I-1b) and their mixtures, and also the compounds of formula (III) are selected from among rimocidin C (IIIa), CE-108C (IIIb) and their mixtures.

In the sense used in this description, the expression "pharmaceutically acceptable excipient" refers to those substances, or combination of substances, known in the pharmaceutical sector, used in the preparation of pharmaceutical forms of administration and including adjuvants, solids or liquids, solvents, surfactants, etc.

If wished, said pharmaceutical composition can furthermore contain one or more therapeutic agents which might possibly strength the therapeutic action of said compounds of formula (I) for example, the compounds rimocidin B and/or CE-108B, rimocidin C, CE-108C and their mixtures, or increase their spectrum of action.

Said pharmaceutical composition can be used for preventing and/or treating infections caused by pathogenic organisms of human or animals which possess a cell membrane containing ergosterol, for example, parasites and fungi that are pathogens of humans or animals.

Therefore, in a specific embodiment, said pharmaceutical composition is an antiparasite composition and can be used in the prevention and/or treatment of infections caused by pathogenic organisms of human or animals which possess a cell membrane containing ergosterol, for example, *Trypanosoma, Leishmania,* etc. If wished, said antiparasite composition can furthermore contain one or more antiparasite agents which might possibly strength the therapeutic action of said compounds of formula (I) for example, the compounds rimocidin B, CE-108B, rimocidin C, CE-108C or which increase their spectrum of action.

In another specific embodiment, said pharmaceutical composition is an antifungal composition and can be used in the prevention and/or treatment of infections caused by fungi (whose cell membranes contain ergosterol). If wished, said antifungal composition can furthermore contain one or more antifungal agents which might possibly strengthen the therapeutic action of compounds of formula (I) for example, rimocidin B, CE-108B, rimocidin C, CE-108C or which increase their spectrum of action. Illustrative, though not limiting, examples of said antifungal agents include polyenes, such as amphotericin B, nystatin, AB-400, allylamines (e.g., terbinafine, nafthafine, etc.), amorolfine, tolnaftalate, etc., azoles such as chlotrimazol, miconazol, ketconazol, fluconazol, itraconazol, etc., benzofurans, for example griseofilvin, etc., pyrimidines, for example fluocytosin, etc.

The compound of formula (I), and among them the compounds of formula (I-1) and (III), will be present in the pharmaceutical composition in a therapeutically effective quantity, in other words, a quantity suitable for exerting its therapeutic effect. In a particular embodiment, the pharmaceutical composition provided by this invention contains between 0.01% and 99.99% by weight of a compound of formula (I), such as a compound selected from among the compounds rimocidin B (I-1a), CE-108B (I-1b), rimocidin C (IIIa) and CE-108C (IIIb) and their mixtures, and can be presented in any suitable pharmaceutical form of administration depending on the chosen administration route, for example, oral, parenteral or topical. A review of the different pharmaceutical forms of administration of drugs and their preparation methods can be found in, for example, Tratado de Farmacia Galénica, C. Faulí i Trillo, 1st edition, 1993, Luzán 5, S. A. de Ediciones.

Therefore the invention also relates to the use of a compound of formula (I) and/or (III) in the preparation of a medicine for the prevention and/or treatment of infection caused by pathogenic fungi of humans or animals whose cell membranes contain ergosterol, for example human or animal parasites or pathogenic fungi of humans or animals. In a particular embodiment, said compound of formula (I) and/or (III) is selected from among rimocidin B (I-1a), CE-108B (I-1b), rimocidin C (IIIa), CE-108C (IIIb) and their mixtures.

Likewise, in another aspect, the invention also provides a method for preventing and/or treating infections caused by pathogenic organisms of human or animals which possess a cell membrane containing ergosterol, for example, parasites and fungi that are pathogens of humans or animals, which comprises the stage of administering a therapeutically effective quantity of a pharmaceutical composition provided by this invention to an animal or human being in need of treatment.

### 2.- Agricultural applications

Another important sector in which the compounds of formula (I), and among them compounds of formula (I-1) or (III), find application is in the agricultural sector. In this regard, the invention provides an antifungal composition useful for controlling infection caused by phytopathogenic fungi (such as *Botrytis cinerea, Fusarium oxysporum, Rhizoctonia solana, Rhizoctonia meloni, Ustilago maydis,* among others) whose cell membranes contain ergosterol, which comprises a compound of formula (I) and/or (III), such as a compound selected from among the compounds rimocidin B (I-1a), CE-108B (I-1b), rimocidin C (IIIa), CE-108C (IIIb) and their mixtures, optionally together with one or more agriculturally acceptable inert vehicles, for preventing or controlling fungal infections under conditions of storage of agro-alimentary products (post-harvest infections), caused by various fungi whose cell membranes contain ergosterol.

As used here, the term "control" includes the inhibition, reduction or halting of the germination of the spores and/or of the growth of the mycelium of fungi which can result in the elimination of said fungi or in the lessening of the damage caused by them.

The expression "agriculturally acceptable inert vehicle" as used here refers to those substances, or combination of substances, known in the agricultural sector, used for vehicularising compounds of interest and including adjuvants, solids or liquids, solvents, surfactants, etc.

In a particular embodiment, said antifungal composition includes, in addition to said compound of formula (I) or (III), a compound selected from among rimocidin B (I-1a), CE-108B (I-1b), rimocidin C (IIIa) and CE-108C (IIIb) and their mixtures, one or more compounds with antifungal activity, for example, one or more compounds which alter the cell membrane of the fungi, or one or more compounds which inhibit the synthesis of ergosterol, such as the aforementioned compounds, or one or more compounds with enzymatic activities required for the modification or degradation of cell walls, for example, one or more lytic enzymes capable of modifying or degrading cell walls of fungi, such as enzymes with cellulolytic, mananolytic, chitinolytic or proteolytic activity (e.g., cellulases, α-(1,3)-glucanases, β-(1,6)-glucanases β-(1,3)-glucanases, mananases, endo- or exo-chitinases, chitosanases, proteases, α- or β-manosidases, etc.).

The compound of formula (I) and/or formula (III) will be present in the antifungal composition in an effective antifungal quantity, in other words, a quantity suitable for controlling the infection caused by phytopathogenic fungi. In a particular embodiment, the useful antifungal composition provided by this invention contains between 0.01% and 100% by weight of said compound of formula (I) and/or (III), for example of said compounds rimocidin B (I-1a), CE-108B (I-1b), rimocidin C (IIIa) and CE-108C (IIIb). Said composition can be prepared by conventional methods and can be presented in liquid or solid form, for example, in granulated form. In addition, said composition can contain additives, for example, preserving agents and stabilisers that will prolong the preservation and stability of it.

Said antifungal composition can, for example, be used for controlling infections caused by phytopathogenic fungi in plants and/or in fruits. Therefore, the invention provides a method for controlling infection caused by a phytopathogenic fungus in a plant, in particular a phytopathogenic fungus whose cell membrane contains ergosterol, which comprises applying said composition to said plant, or to the medium surrounding it, in order to control the phytopathogenic fungus. In a particular embodiment, said method comprises the application of said composition, in a suitable quantity, to the aerial parts of the plant with the aim of preventing and/or treating a fungal infection caused by a phytopathogenic fungus.

The invention also provides a method for controlling infection caused by a phytopathogenic fungus in a fruit, in particular a phytopathogenic fungus whose cell membrane contains ergosterol, which comprises applying said composition to said fruit in order to control the phytopathogenic fungi. In a particular embodiment, the application of said composition, in a suitable quantity, to the fruit is done prior to its picking (pre-harvest) while in another alternative embodiment, the application of the composition is done on the already picked collected fruit (post-harvest).

### 3.- Agro-alimentary applications

The compounds of formula (I) and/or (III), and in particular rimocidin B (I-1a), CE-108B (I-1b), rimocidin C (IIIa), CE-108C (IIIb), also have application in the agro-alimentary sector. In this regard, the invention provides an antifungal composition useful for controlling fungi that might possibly develop on prepared foods, for example, on the surface of prepared foods, such as dairy produce, for example, cheeses etc., which comprises a compound of formula (I), such as a compound selected from among the compounds rimocidin B (I-1a), CE-108B (I-1 b), rimocidin C (IIIa), CE-108C (IIIb) and their mixtures, optionally together with one or more acceptable inert vehicles from the agro-alimentary point of view, such as liposome suspensions, suspensions in water, etc., among others.

The term "control" includes the inhibition, reduction or halting of the germination of the spores and/or of the growth of the mycelium of fungi which can result in a notable lessening of the damage caused by them; in this way, the spoiling of food caused by the growth of fungi can be prevented or treated.

The terms "acceptable inert vehicle from the agro-alimentary point of view" refers to those substances, or combination of substances, known in the agro-aiimentary sector, used for vehicularising compounds of interest and including adjuvants, solids or liquids, solvents, surfactants, etc.

In a particular embodiment, said antifungal composition includes, in addition to said compound of formula (I) a compound selected from among rimocidin B (I-1a), CE-108B (I-1b), rimocidin C (IIIa) and CE-108C (IIIb) and their mixtures, one or more compounds with antifungal activity, for example, one or more compounds which alter the cell membrane of the fungi, or one or more compounds which inhibit the synthesis of ergosterol, such as the aforementioned compounds, or one or more compounds with enzymatic activities required for the modification or degradation of cell walls, for example, one or more lytic enzymes capable of modifying or degrading cell walls of fungi, such as enzymes with cellulolytic, mananolytic, chitinolytic or proteolytic activity (e.g., cellulases, α-(1,3)-glucanases, β-(1,6)-glucanases β-(1,3)-glucanases, mananases, endo- or exo-chitinases, chitosanases, proteases, α- or β-manosidases, etc.).

The compound of formula (I) will be present in the antifungal composition in an effective antifungal quantity, in other words, a quantity suitable for controlling the infection caused by fungi liable to develop on prepared foods. In a particular embodiment, said the antifungal composition useful for controlling the infection caused by fungi liable to develop on prepared foods provided by this invention contains between 0.01% and 100% by weight of said compound of formula (I), for example of said compounds rimocidin B (I-1a), CE-108B (I-1b), rimocidin C (IIIa) and CE-108C (IIIb). Said composition can be prepared by conventional methods and can be presented in liquid or solid form, for example, in granulated form. In addition, said composition can contain additives, for example, preserving agents and stabilisers that will prolong the preservation and stability of it.

Said antifungal composition can, for example, be used for controlling fungi liable to develop on prepared foods, for example, on the surface of prepared foods. Therefore, the invention also provides a method for controlling infection caused by a fungus liable to develop on prepared foods, in particular for controlling infection caused by a fungus liable to develop on prepared foods whose ceii membrane contains ergosterol, which comprises applying said antifungal composition to said prepared food, or to the medium surrounding it, in order to control the fungus liable to develop on prepared foods. In a particular embodiment, said method comprises the application of said composition, on the surface of the prepared food with the aim of preventing and/or treating the spoiling of food caused by the growth of fungi. Said antifungal composition is applied externally to the surface of the prepared food.

### Method for obtaining novel amidated polyenes

Compounds of formula (I) and among them compounds of formula (I-1), and more particularly the compounds rimocidin B (I-1a) and/or CE-108B (I-1b), are present in the fermentation culture of recombinant micro-organisms identified in this description as *Streptomyces diastaticus* var. 108/743B and *Streptomyces diastaticus* var. 108/784, obtained by transformation of *Streptomyces diastaticus* var. 108 (Pérez-Zúñiga F.J., et al. (2004), J. Antibiot. (Tokyo) 57: 197-204), with the plasmids identified in this description as pSM743B and pSM784, respectively, or of the recombinant micro-organism identified in this description as *Streptomyces diastaticus* var. 108::PM1-500/743B obtained by transformation of *Streptomyces diastaticus* var. 108/PM1-500 (Seco E.M., et al., 2004, Chem. Biol. 11: 357-366), with the plasmid identified as pSM743B, as described in Example 1 accompanying this description and included in Table 1. Said compounds can be obtained directly from those fermentation cultures and easily purified using relatively simple conventional methods, for example, by means of the use of ion exchange columns and/or hydrophobic interaction or reverse phase columns.

The plasmid pSM743B (Table 1, Example 1) (Figure 1A) derives from the plasmid plJ922 and includes the gene *rimA* and the erythromycin resistance gene *(ermE).* The plasmid pSM784 (Table 1, Example 1) (Figure 1 B) derives from the plasmid plJ941 and contains and the erythromycin resistance gene *(ermE).*

Said plasmids plJ922 and plJ941 (Lydiate D.J. et al., 1985, Gene 35: 223-235) are vectors derived from the replicon SCP2*, a plasmid with a low number of copies coming from *Streptomyces coelicolor.*

Therefore, in another aspect, the invention relates to a method for the production of a compound of formula (I-1) which comprises cultivating a micro-organism selected from among *Streptomyces diastaticus* var. 108/784, *Streptomyces diastaticus* var. 108/743B, *Streptomyces diastaticus* var. 108::PM1-500/743B, and combinations of them, under conditions that permit the production of compounds of formula (I) and, if wished, to isolate and purify those compounds. In a particular embodiment, the compounds of formula (I) are selected from between rimocidin B (I-1a) and CE-108B (I-1b) and their mixtures.

As mentioned earlier, the formation of the amide- group in polyenes is due to an "adornment" activity on account of an amidotransferase. By means of *in vitro* amidation trials with cell-free extracts of producers of amidated polyenes and using rimocidin (IIa) and CE-108 (IIb) as substrates, it has been possible to conclude that the formation of the amide- group is in fact due to an ATP/Mg⁺⁺ dependent amidotransferase activity and capable of using glutamine as the donor of amide- groups (see Example 2, section B). The same trials conducted with cell-free extracts of *Streptomyces* sp. RGU5.3 has permitted it to be confirmed that AB-400 (IVb), the amide of pimaricin (IVa), originates by means of an amidotransferase activity very similar to that of *S. diastaticus* var. 108, which acts on the free carboxyl- group of pimaricin (IVa) in order to transform it into an amide- group in a reaction that is also ATP/Mg⁺⁺ dependent.

Moreover, it has furthermore been possible to conclude that the amidotransferase activity present in cell-free extracts of the genetic recombinants of *S. diastaticus* var. 108 presents a relaxed specificity towards the substrate being capable of modifying not only the native substrates rimocidin (IIa) and CE-108 (IIb) in their corresponding amides rimocidin B (I-1a) and CE-108B (I-1b) but it is also capable of recognising heterologous substrates such as pimaricin (IVa). This did not occur for the amidotransferase activity of *Streptomyces* sp. RGU5.3, which was only capable of recognising pimaricin (IVa) as a substrate under the tested conditions.

The free carboxyl- group is fairly well preserved in the majority of typical polyenes, such as amphotericin B, nystatin, pimaricin (IVa), candicidin, etc. The substitution of these carboxyl- group for amide- groups would probably give rise to amidated polyenes with improved pharmacological properties such as greater solubility in water, greater antifungal activity, and lower haemolytic activity. In short, the compounds would present greater selective toxicity towards fungi.

Therefore, in this aspect, the invention provides an enzymatic method, hereinafter the inventive enzymatic method, for obtaining an amidated polyene from polyenes with free carboxylated groups in the macrolactone ring using cell-free extracts of producing strains of amidated polyenes by means of the following stages:
a) set up a mixture with a substrate consisting of a polyene with free carboxylated groups or mixtures of several of them, purified or not, and a protein extract coming from a producing strain or strains of amidated polyenes, and
b) reaction of the mixture of a) under conditions: of ATP/Mg⁺⁺ dependence and capable of using glutamine as donor of amide- groups, and
c) purification of amidated polyenes.

A particular object of the invention consists of the inventive enzymatic method in which the amidated polyene to obtain is CE-108B (I-1b), rimocidin B (I-1a) or their mixtures, the substrate polyene of a) is CE-108 (IIb) rimocidin (IIa) or their mixtures, purified or not, and in which the extract of a) is obtained from the following strains: *S. diastaticus* var. 108/784 and *S. diastaticus* var. 108/743B.

Another particular object of the invention consists of the inventive enzymatic method in which the amidated polyene to obtain is AB-400 (IVb), the substrate polyene of a) is pimaricin (IVa), purified or not, and in which the extract of a) is obtained from the following strains: *S. diastaticus* var. 108/784 and *S. diastaticus* var. 108/743B.

Another particular object of the invention consists of the inventive enzymatic method in which the amidated polyene to obtain is AB-400 (IVb), the substrate polyene of a) is pimaricin (IVa), purified or not, and in which the extract of a) is obtained from the strain *Streptomyces* sp. RGU5.3.

In another aspect, the invention relates to cell-free extracts of producers of amidated polyenes, carriers of an amidotransferase activity, capable of converting *"in vitro"* carboxylated polyenes into their corresponding amides necessary for commencing the inventive enzymatic method.

Cell-free extracts are understood to be those extracts coming from the homogenisation of cells by various mechanical methods (habitually applied in the field of handling proteins) and the later fractionating either by filtration or differential centrifugation in order to have a clarified product containing most of the cell components, either in suspension or in solution.

In another particular aspect, the invention relates to the cell-free extracts of the micro-organisms *S. diastaticus* var. 108/743B, *S. diastaticus* var. 108/784 (DSM 17187) - both of them producers of the amidated polyenes CE-108B (I-1b) and rimocidin B (I-1a) - carriers of an amidotransferase activity with the capacity for converting rimocidin (IIa) and CE-108 (IIb) as well as other heterologous substrates into their corresponding amides; and the cell-free extract of the micro-organisms *Streptomyces* sp. RGU5.3, the producer of the amidated polyene AB-400 (IVb), capable of converting at least pimaricin (IVa) into its corresponding amidated polyene AB-400 (IVb) under the tested conditions.

Alternatively, the inventive enzymatic method can be carried out using conventional methods of immobilised cell-free enzymatic systems (inventive cell-free extract) on solid supports, causing the components of the reaction with the corresponding mobile phases to flow, following the technology of the field of immobilised systems known to a average technician in the sector.

### Method for obtaining novel methylated polyenes

Compounds of formula (III) and in particular the compounds rimocidin C (IIIa) and CE-108C (IIIb), are present in the fermentation culture of the recombinant micro-organism identified in this description as *Streptomyces diastaticus* var. 108::PM1-768/743B obtained by disruption of the gene *rimG* by means of homologous recombination with the recombinant phage PM1-768 (see Table 1, Example 2) and by the transfer of the plasmid pSM743B by conjugation from the strain *Streptomyces diastaticus* var. 108/743B, as contained in Table 1, Example 1. Both the fragment for generating the disruption of the gene *rimG* and the fragment contained in the complete gene *rimA* for avoiding polar effects can be easily obtained by persons with a certain skill in the field, using conventional techniques of amplification of deoxyribonucleic acid (DNA). For the corresponding amplifications the strain deposited in Deutsch Sammlung von Mikroorganismen und Zellkulturen GmbH (DSMS), Braunschweig, Germany (access number DSM 17187) can be used. The corresponding vectors can be introduced using conventional techniques (transformation, transfection, conjugation, etc.) in the strain DSM 17187 mentioned above.

In this invention, the recombinant phage PM1-768 was obtained from the phage PM 1, on which a fragment was cloned internal to the gene *rimG* and the promoter *ermEₚ* was introduced (a promoter widely used in the handling of *Streptomyces* for gene expression: Kieser et al. 2000, Practical Streptomyces genetics, The John Innes Foundation, Norwich, UK), with the idea of avoiding a polar effect on genes located downstream; the intermediate clones as far as reaching the final construction are described in Table 1 of Example 2 and can be easily obtained by persons with a certain skill in working with *Streptomyces,* as stated above.

Said compounds rimocidin C (IIIa) and CE-108C (IIIb) can be obtained directly from the fermentation culture of the micro-organism *Streptomyces diastaticus* var. 108::PM1-768/743B and easily purified by relatively simple conventional methods, for example, by means of using hydrophobic interaction or reverse phase columns.

Therefore, in another aspect, the invention relates to a method for the production of a compound of formula (III) which comprises the following stages:
- culture of the micro-organism *Streptomyces diastaticus* var. 108::PM1-768/743B under conditions that permit the production of compounds of formula (III)
- obtaining the fermentation culture and, if wished,
- the isolation and purification of those compounds formula (III).

In a particular embodiment, the method is carried out in order to obtain the compounds of formula (III) which are selected from among rimocidin C (IIIa), CE-108C (IIIb) and their mixtures.

The culture medium for that recombinant micro-organism generally consists of one or more sources of carbon, one or more sources of nitrogen, one or more inorganic salts that can be assimilated by the micro-organism, and, if necessary, one or more nutrients such as vitamins and amino acids, dissolved in an aqueous medium. Said media, as with the appropriate conditions (aeration/stirring, temperature and fermentation time, stages, etc.) are known to experts in the field. Illustrative examples, though without being limiting, of the media and conditions for growing these recombinant micro-organisms are mentioned in Example 2 of the invention (section "Experimental methods").

The fermentation culture of said recombinant micro-organisms *Streptomyces diastaticus* var. 108::PM1-768/743B or their functional equivalents comprise a compound selected from between a compound of formula (III), for example, rimocidin C (IIIa), CE-108C (IIIb) and their mixtures, and can be used as such or they can be subsequently treated in order to separate the compound of interest, which can be isolated by conventional methods. By way of illustration, in a particular embodiment, the cell culture is centrifuged in order to separate the supernatant and the cell extract and the supernatant us used for isolating and, if wished, purifying the polyene of interest, either amidated or non-amidated. The study of the physical-chemical characteristics of those compounds permits an average technician to design a method for their purification starting from the supernatant.

### Recombinant micro-organisms and applications

### 1. Recombinant micro-organisms involved in the obtaining of amidated polyenes and applications.

The recombinant micro-organisms *Streptomyces diastaticus* var. 108/743B, *Streptomyces diastaticus* var. 108/784 and *Streptomyces diastaticus* var. 108::PM1-500/743B form part of the present invention. Therefore, in another aspect, the invention is related to a micro-organism selected from among *Streptomyces diastaticus* var. 108/743B, *Streptomyces diastaticus* var. 108/784 and *Streptomyces diastaticus* var. 108::PM1-500/743B and provides a culture of a micro-organism selected from among *Streptomyces diastaticus* var. 108/743B, *Streptomyces diastaticus* var. 108/784 and *Streptomyces diastaticus* var. 108::PM1-500/743B and combinations of them.

Likewise, the invention relates to the use of a micro-organism selected from among *Streptomyces diastaticus* var. 108/743B, *Streptomyces diastaticus* var. 108/784 and *Streptomyces diastaticus* var. 108::PM1-500/743B and combinations of them, or of said culture of micro-organisms selected from among *Streptomyces diastaticus* var. 108/743B, *Streptomyces diastaticus* var. 108/784 and *Streptomyces diastaticus* var. 108::PM1-500/743B and combinations of them, in obtaining a compound of formula (I); preferably compounds of formula (I-1). In a particular embodiment, the compound of formula (I-1) is selected from between rimocidin B (I-1a), CE-108B (I-1b) and their mixtures.

Moreover, the trials conducted by the inventors revealed that the fermentation culture of said recombinant micro-organisms *Streptomyces diastaticus* var. 108/743B, *Streptomyces diastaticus* var. 108/784 and *Streptomyces diastaticus* var. 108::PM1-500/743B also contains, in addition to the amidated polyenes rimocidin B (I-1a) and/or CE-108B (I-1b), the non-amidated polyenes rimocidin (IIa) and/or CE-108 (IIb), which contain a free carboxyl- group.

Therefore, in another aspect, the invention relates to a method for the production of a compound selected from among a compound of formula (I), rimocidin (IIa), CE-108 (IIb) and their mixtures, which consists of cultivating a micro-organism selected from among *Streptomyces diastaticus* var. 108/743B, *Streptomyces diastaticus* var. 108/784, *Streptomyces diastaticus* var. 108::PM1-500/743B and combinations of them, under conditions which permit the production of a compound selected from among a compound of formula (I), rimocidin (IIa), CE-108 (IIb) and their mixtures, and, if wished, to isolate and purify said compound. In a particular embodiment, the compound of formula (I) selected from among rimocidin B (I-1a), CE-108B (I-1b) and their mixtures.

The fermentation culture of a micro-organism selected from among *Streptomyces diastaticus* var. 108/743B, *Streptomyces diastaticus* var. 108/784, *Streptomyces diastaticus* var. 108::PM1-500/743B and combinations of them, which consists of a compound selected between a compound of formula (I), rimocidin (IIa), CE-108 (IIb) and their mixtures, can be potentially useful as a biocide and constitutes an additional aspect of this invention. In a particular embodiment, the compound of formula (I) is selected from among rimocidin B (I-1a), CE-108B (1-1b) and their mixtures.

The culture medium of said recombinant micro-organisms generally consists of one or more sources of carbon, one or more sources of nitrogen, one or more inorganic salts that can be assimilated by the micro-organism, and, if necessary, one or more nutrients such as vitamins and amino acids, dissolved in an aqueous medium. Said media, as with the appropriate conditions (aeration/stirring, temperature and fermentation time, strains, etc.), are known to experts in the field. Illustrative examples, though without being limiting, of the media and conditions for growing these recombinant micro-organisms are mentioned in the Example (section "Experimental methods"). The fermentation culture of said recombinant micro-organisms selected from among *Streptomyces diastaticus* var. 108/743B, *Streptomyces diastaticus* var. 108/784, *Streptomyces diastaticus* var. 108::PM1-500/743B and combinations of them comprise a compound selected from between a compound of formula (I), for example, rimocidin B (I-1a), CE-108B (I-1b), rimocidin (IIa), CE-108 (IIb) and their mixtures, and can be used as such or they can be subsequently treated in order to separate the compound of interest, which can be isolated by conventional methods. By way of illustration, in a particular embodiment, the cell culture is centrifuged in order to separate the supernatant and the cell extract and the supernatant us used for isolating and, if wished, purifying the polyene of interest, either amidated or non-amidated. The study of the physical-chemical characteristics of those compounds permits an average technician to design a method for their purification starting from the supernatant.

### 2. Recombinant micro-organisms involved in the obtaining of methylated polyenes and applications.

The micro-organism *Streptomyces diastaticus* var. 108::PM1-768/743B or any other functional equivalent forms part of the present invention. Therefore, in another aspect, the invention is related to the micro-organism *Streptomyces diastaticus* var. 108::PM1-768/743B or functional equivalents useful for carrying out the method for obtaining the compounds of formula (III) and providing a culture of those micro-organisms. A particular embodiment of the invention consists of the micro-organism *Streptomyces diastaticus* var. 108::PM1-768/743B (deposit number: DSM 17482) which is the producer of the methylated polyenes rimocidin C (IIIa) and CE-108C (IIIb).

As used in the present invention, the term "functional equivalent" refers to an element - be it a micro-organism, vector, gene construction, or gene fragment, method of disruption of a gene, of genetic transformation of a cell or micro-organism, depending on the case - which can be developed by average technicians in the field with the different existing alternatives and which possesses identical or homologous characteristics to the original element being referred to.

So, in this context, and by way of illustration, "functional equivalent/s" are understood to be those recombinant micro-organisms which can be obtained either by chromosome deletion of the gene *rimG,* instead of inactivating insertion or with any other technique: said deletion can be easily performed by means of using conventional vectors, either non-replicative plasmids or replicable plasmids having their origin in heat-sensitive replication. These deletions or insertions can be carried out by a technician with a certain experience in the field.

Likewise, the invention relates to the use of the micro-organisms *Streptomyces diastaticus* var. 108::PM1-768/743B or its functional equivalents in order to obtain a compound of formula (III). In a particular embodiment, the compound of formula (III) is selected from among rimocidin C (IIIa), CE-108C (IIIb) and their mixtures.

For certain applications, the fermentation culture thus obtained with the inventive method can be used directly for preparing certain biocide solutions, without any need to purify the compounds of the present invention. So, the fermentation culture of the micro-organisms *Streptomyces diastaticus* var. 108::PM1-768/743B or its functional equivalents stated above, which comprise a compound of formula (III), can be potentially useful as a bioicide and constitutes an additional aspect of this invention.

### Vectors and applications

### 1. Vectors involved in the induction of amidated polyenes and applications

When the plasmid pSM784, derived from the vector plJ941 on which the erythromycin resistance gene has been cloned, is introduced into *S. diastaticus* var. 108, it gives rise to a strain (*S. diastaticus* var. 108/784) which is the producer of the novel amidated polyenes rimocidin B (I-1a) and CE-108B (I-1b) as well as of rimocidin (IIa) and CE-108 (IIb).

Moreover, when the plasmid pSM743B, derived from the vector SCP2* plus the gene *ermE* and carrier, furthermore, of a fragment of the cluster *rim* (which contains the gene *rimA*) expressed under an endogenous or exogenous promoter, such as the promoter *xysA* (*xysA*p) of the gene of the xylanase of *Streptomyces halstedii* JM8 (Ruiz-Arribas A., et al., 1997, Appl. Environ. Microbiol, 63: 2983:2988), is introduced into *S. diastaticus* var. 108 or into *Streptomyces diastaticus* var. 108::PM1-500, an increase is observed in the total production both of amidated polyenes [rimocidin B (I-1a) and CE-108B (I-1b)] and of non-amidated polyenes [rimocidin (IIa) and CE-108 (IIb)]. This fact could be used for increasing the total production of polyenes with carboxyl-group and/or amidated polyenes in other producing organisms of said polyenes.

Therefore, in another aspect, the invention relates to a vector, herein after the inventive vector, selected from among:
a) a vector derived from the vector SCP2*, or a fragment of it, which contains the replication origin of SCP2* and the erythromycin resistance gene *ermE*);
b) a vector which contains (i) the replication origin of the vector SCP2*; (ii) the gene *ermE,* and (iii) a fragment of the vector SCP2*;
c) a vector which contains (i) a replication origin, (ii) the gene *ermE,* and (iii) a fragment of the vector SCP2*, i which said replication origin is different from the replication origin of SCP2*;
d) a vector which lacks a replication origin and contains the gene *ermE,* and a fragment of the vector SCP2*;
e) a vector derived from the vector SCP2*, which contains (i) a replication origin, (ii) the gene *ermE*; and (iii) the entire biosynthetic cluster of a polyene or a fragment of said cluster;
f) a vector derived from the vector SCP2*, which contains (i) a replication origin, equal to or different from the replication vector SCP2 (ii) the gene *ermE;* (iii) a fragment of the vector SCP2*; and (iv) the entire biosynthetic cluster of a polyene or a fragment of said cluster;
g) a vector which lacks a replication origin and contains the gene *ermE,* and the entire biosynthetic cluster of a polyene or a fragment of said cluster; and
h) a vector which lacks a replication origin and contains (i) the gene *ermE;* (ii) a fragment of the vector SCP2*; and (iii) the entire biosynthetic cluster of a polyene or a fragment of said cluster.

Practically any vector derived from the vector SCP2* can be used, for example, pIJ922, pIJ941, etc. The gene *ermE* is a known gene (Uchiyama et al., (1985) Gene 38: 103-110). As used here, the expression "fragment of the vector SCP2*" refers to a nucleic acid that consists of one or more fragments of SCP2* sufficient for inducing the formation of amidated polyenes, Trials conducted by the inventors have revealed that a nucleic acid consisting of one or more fragments of the vector SCP2*, together with the gene *ermE,* is necessary and sufficient so that the amidated polyenes can be generated in the recombinant micro-organisms.

In a particular embodiment, the inventive vector is a replicative vector derived from SCP2*, which contains the replication origin of SCP2*, and is also the carrier of the gene *ermE,* for example, pSM784. Said plasmid can be introduced by conventional methods (e.g., transformation, electroporation, conjugation, etc.) in producing micro-organisms of polyene macrolides containing a free carboxyl- group (e.g., amphotericin B, nystatin, pimaricin, candicidin, etc.) with the aim of producing the corresponding amidated polyenes. Illustrative examples, though without being limiting, of such micro-organisms include *S. noursei, S. albidus, S. rimosus, S. nodosus, S. natalensis, S. chattanoogensis, S. griseus,* etc.

In another particular embodiment, the inventive vector is a replicative vector containing the replication origin of SCP2*, the gene *ermE,* and a fragment of the vector SCP2*.

In another particular embodiment, the inventive vector is a replicative vector containing the replication origin different from the replication origin of SCP2*, the gene *ermE,* and a fragment of the vector SCP2*.

In another particular embodiment, the inventive vector is an integrative or non-replicative vector lacking the replication origin and containing the gene *ermE,* and a fragment of the vector SCP2*.

In another particular embodiment, the inventive vector is a replicative vector which contains a replication origin equal to or different from the replication origin of SCP2*, the gene *ermE,* and the entire biosynthetic cluster of a polyene or a fragment of said cluster.

In another particular embodiment, the inventive vector is a replicative vector which contains the replication origin of SCP2*, the gene *ermE,* a fragment of the vector SCP2*.and the entire biosynthetic cluster of a polyene or a fragment of said cluster.

In another particular embodiment, the inventive vector is an integrative or non-replicative vector lacking the replication origin and containing the gene *ermE,* and the entire biosynthetic cluster of a polyene or a fragment of said cluster.

In another particular embodiment, the inventive vector is an integrative or non-replicative vector lacking the replication origin and containing the gene *ermE,* a fragment of the vector SCP2* and the entire biosynthetic cluster of a polyene or a fragment of said cluster.

When inventive vectors are used containing the entire biosynthetic cluster of a polyene or a fragment of it for transforming producing micro-organisms of polyenes with free carboxyl- groups, the production of amidated polyenes and/or an increase in the total production of both amidated polyenes and non-amidated polyenes is observed, due to which said vectors can be used for increasing the total production of polyenes with free carboxyl- groups and/or amidated polyenes in producing organisms of polyenes.

Practically any biosynthetic cluster or fragment thereof can be present in the inventive vector; nevertheless, in a specific embodiment, said vector comprises the entire biosynthetic cluster *rim* of rimocidin (Seco E.M., et al., 2004, Chem. Biol. 11: 357-366). Although any fragment of the cluster *rim* can be used, in a specific embodiment, said fragment of the cluster *rim* includes the gene *rimA* of the cluster *rim.*

Said biosynthetic cluster of a polyene or fragment thereof can optionally be found under the control of a promoter (in other words, operatively joined to said promoter in such a way that it directs the expression of the gene it controls). Said promoter can be endogenous or exogenous. Although practically any exogenous promoter functional in the micro-organism to transform later on with that vector could be used, in a particular embodiment, said exogenous promoter is a promoter functional in *Streptomyces* sp. such as the promoter *xysA* (*zysA*p) of the gene of the xylanase of *Streptomyces halstedii* JM8 (Ruiz-Arribas, A., et al., 1997, Appl. Environ. Microbiol, 63: 2983-2988). The plasmid pSM743B constitutes an illustrative example of this type of inventive vector.

The inventive vectors can be obtained by conventional methods known to experts in the field (Sambrook et al., Molecular Cloning, A Laboratory Manual, Cold Spring Harbor, NY, 1989).

The inventive vectors can be used for generating amidated or non-amidated polyenes in producing micro-organisms of vectors which contain a free carboxyl- group. Likewise, combinations of vectors containing, on the one hand, (i) a vector which comprises a biosynthetic cluster of a polyene, such as the cluster *rim,* or a fragment of it, and, on the other hand, (ii) a vector derived from SCP2* which comprises the gene *ermE* or a vector which comprises a replication origin different from that of SCP2*, the gene *ermE,* and a fragment of the vector SCP2*, can be introduced into producing micro-organisms of polyene macrolides in order to generate amidated or non-amidated polyenes in said micro-organisms.

Therefore, in another aspect, the invention relates to the use of an inventive vector for being introduced, by conventional methods, into polyene macrolides containing free carboxyl- groups (e.g., amphotericin B, nystatin, rimocidin, pimaricin, candicidin, etc.) with the aim of producing the corresponding amidated polyenes starting from the corresponding generated recombinant micro-organisms. Among the micro-organisms suitable for use as hosts of the inventive vectors are to be found producing micro-organisms of polyenes presenting a free carboxyl- group such as *Streptomyces nodosus,* a natural producer of amphotericin B, *Streptomyces rimosus,* a producer of rimocidin, *Streptomyces griseus,* a producer of candicidin; *Streptomyces natalensis,* a producer of pimaricin; *Streptomyces noursei,* a producer of nystatin, etc.

In another aspect, the invention relates to a method for obtaining recombinant producing micro-organisms of polyene macrolides containing an amide- group which consists of introducing an inventive vector into producing micro-organisms of polyene macrolides containing free carboxyl- groups. Alternatively, said recombinant producing micro-organisms of polyene macrolides containing an amide- group can be obtained by introducing into producing micro-organisms of polyene macrolides containing free carboxylgroups combinations of, on the one hand, (i) a vector which comprises a biosynthetic cluster of a polyene, such as the cluster *rim,* or a fragment of it, and, on the other hand, (ii) a vector derived from SCP2* which comprises the gene *ermE* or a vector which comprises a replication origin different from that of SCP2*, the gene *ermE,* and a fragment of the vector SCP2*, can be introduced into producing micro-organisms of polyene macrolides containing free carboxylgroups in order to generate amidated or non-amidated polyenes in said micro-organisms. The introduction of said inventive vectors or combinations of vectors into said micro-organisms can be carried out by conventional techniques known to experts in the field, e.g., transformation, electroporation, conjugation, etc. Illustrative examples, without being limiting, of producing micro-organisms of polyene macrolides containing free carboxyl- groups include various species of *Streptomyces,* for example, *S. noursei, S. rimosus, S. nodosus, S., natalensis, S. griseus,* etc.

The recombinant micro-organisms, obtained as stated above, hereinafter the inventive recombinant micro-organisms, form part of the present invention and constitute an additional aspect thereof.

In another aspect, the invention relates to a method for producing a polyene macrolide which comprises cultivating an inventive recombinant micro-organism under conditions that permit the production of said polyene macrolide and, if wished, to isolate and purify that compound. In a particular embodiment, said polyene macrolide is selected from among a polyene macrolide containing a free carboxyl- group, a polyene macrolide containing an amide- group and their mixtures. Among said polyene macrolides containing an amide- group are to be found the compounds AB-400 (IVb) and the compounds of formula (I-1), for example, the compounds rimocidin B (I-1a) and CE-108B (I-1b), and their mixtures. Illustrative examples, though without being limiting, of polyene macrolides that can be obtained according to the method mentioned above include compounds of formula (I), for example, the compounds selected from among pimaricin (IVa), AB-400 (IVb), rimocidin (IIa), rimocidin B (I-1a), CE-108 (IIb), CE-108B (I-1b) and their mixtures.

Said recombinant micro-organisms will be cultivated in any suitable medium for the fermentation of those micro-organisms and they will in general contain one or more sources of carbon, one or more sources of nitrogen, one or more inorganic salts that can be assimilated by the micro-organism, and, if necessary, one or more nutrients such as vitamins and amino acids, dissolved in an aqueous medium, and the appropriate conditions will be applied (aeration/stirring, temperature and fermentation time, stages, etc.) for the growth of the micro-organisms and the production of the polyene macrolides. The polyene macrolides obtained are advantageously secreted into the culture medium from where they can be recovered by means of conventional techniques, for example, by means of using chromatographic methods, optionally prior to withdrawal of the cell extract. The polyene macrolide/s of interest can be separated on the basis of its/their physical-chemical characteristics, which permits a method to be designed for its/their purification starting from the culture medium following the fermentation process.

### 2. Vectors involved in the induction of methylated polyenes and applications

The invention also relates to the use of the recombinant phage PM1-768B, derived from the actinophage PM1 (Malpartida and Hopwood (1986) Mol. Gen. Genet. 205: 66-73) on which a fragment of DNA internal to the gene rimG has been cloned together with the promoter *ermEₚ;* the promoter in front of the fragment internal to the gene avoids possible polar effects on the genes located following the insertion point. Alternatively, a fragment of DNA could be used containing another promoter activity functional for *Streptomyces,* cloned in front of any fragment internal to the gene rimG obtained by amplification of the chromosome DNA of the producer of rimocidin (IIa) and CE-108 (IIb); the resulting construction can be cloned indifferently in the vector PM1 or in another vector non-replicative in *Streptomyces* (or it is replicative but is subjected to culture conditions where the vector does not replicate, such as plasmids of heat-sensitive replication origin), according to the methodology used in the field and which is accessible to any operator with experience in it. The recombinant strain (*Streptomyces diastaticus* var. 108/PM1-768) (or other functionally equivalent which can be generated by the methods stated above) would have to be the producer of the intermediary compounds of the biosynthetic pathway for rimocidin (IIa) and CE-108 (IIb), since the deactivated insertion will only affect the capacity of the recombinant for completing the oxidation of the methyl- side group introduced by module 7 of the synthetase polyketide of rimocidin (IIa) and CE-108 (IIb) (Seco *et al.* 2004, Chem. Biol, cited earlier). Nevertheless, under these initial conditions, and in a way that is unexpected, the disruption of the gene *rim*G that takes place generated a recombinant incapable of producing polyenes. This is interpreted in the sense that the promoter used for the disruption is incapable of preventing polar effects probably on the gene *rimA* located after the insertion point. For this reason, in order to ensure the absence of polar effects on the gene *rimA,* the disruptant has to be complemented with the plasmid pSM743B or other functionally equivalent vector, capable of complementing a disruption of the gene *rimA* in the chromosome. The genetic recombinant thus obtained would contain an additional copy of the gene *rimA* in the plasmid pSM743B (or other functionally equivalent vector) in addition to the chromosome located after the disruption of the gene *rim*G*.* Any polar effect on the chromosomic copy of the gene *rimA* as a consequence of the insertion would be complemented by the extrachromosomic copy cloned in pSM743B (or other functionally equivalent vector), originating a recombinant affected exclusively in the gene *rimG* (oxidation of the side methyl- group of the macrolactone ring). The plasmid pSM743B (or other functionally equivalent vector, as stated above) can be transferred by means of any other technique habitually available for the handling of *Streptomyces* (transformation of protoplasts, infection, conjugation, etc.). Having confirmed the constructions, HPLC analysis of the fermentation culture of the resulting strain (*Streptomyces diastaticus* var. 108::PM1-768/743B or other functionally equivalent as stated above) confirmed the production of rimocidin C (IIIa) and CE-108C (IIIb) but not of the parent tetraenes rimocidin (IIa) and CE-108 (IIb). Optionally, the mutation of the gene *rimG* can be carried out by deletion of a fragment internal to the gene *rimG* following conventional techniques used for the handling of *Streptomyces.*

So, the present invention relates to a method for the obtaining of the inventive producing strain for methylated polyenes *S. diastaticus* var. 108::PM1-768/743B or its equivalents in which the resulting strain is exclusively affected in the expression of the gene rimG and in that it comprises the following stages:
a) Obtaining of a mutant in the gene *rimG* of the micro-organism *S. diastaticus* var. 108 or of its functional equivalents by means of the disruption or deletion of said gene, incapable of producing polyenes, and
b) its later transformation with a vector, preferably a plasmid, capable of complementing the disruption of the gene *rimA* in the chromosome in said mutant.

More specifically, the invention relates to a method for obtaining the inventive micro-organism in which the mutant of step a) is obtained by means of using the recombinant phage PM1-768 or any other deactivating system that is functionally equivalent for generating disruption or deletion of the gene *rimG* of the strain *S. diastaticus* var. 108 and in which step b) is carried out with the plasmid pSM743B (functionally equivalent vector as stated above) in order to complement the possible polar effect of the gene *rimA* in the chromosome of the recombinant.

In addition to the specific embodiment above, the invention relates in another aspect to the interruption of the gene *rimG* in the chromosome of *Streptomyces diastaticus* var. 108 by any conventional method and using any suitable vector. Said interruption will be able to be carried out using a strong promoter for avoiding a polar effect such as might be the promoter *ermE_{P}^{*}* (Kieser et al. (2000) Practical Streptomyces Genetics, The John Innes Foundation, Norwich, UK), which will be responsible for the production of the methylated polyenes rimocidin C (IIIa) and CE-108C (IIIb) directly in the disrupting strain; or complementing the polar effect on the gene *rimA* by mean of the expression of said gene *rimA* under the control of an exogenous promoter using any vector as tool.

Moreover, in another aspect the invention relates to the disruption in other producers of polyenes of the coding gene for the cytochrome P450 monooxygenase involved in this same oxidation (formation of the carboxyl-group starting from the methyl- group of the corresponding polyenes) with the aim of obtaining methylated derivatives of them. It is described that a cytochrome P450 monooxygenase is involved in this oxidation and is found coded in biosynthetic clusters of polyenes described so far (Aparicio et al., 2003, Appl Microbiol Biotechnol 61: 179-188). Some genes involved in this oxidation have been described, and one can cite the genes *pimG* (Aparicio et al., 2000, Chem. Biol. 7: 895-905), *amphN* (Caffrey, et al., 2001, Chem. Biol. 8: 713-723), *nysN* (Brautaset et al., 2000, Chem. Biol. 7: 395-403) and *canC* (Campelo et al., 2000, Microbiology 148: 51-59), for the biosynthesis of pimaricin, amphotericin, nystatin and candicidin respectively. The methylated derivatives of these compounds would present the improved functions of the methylated compounds described in the invention rimocidin C and CE-108C, in other words, less toxicity and greater specificity to fungi and parasites.

Therefore, in another aspect, the invention relates to a method for obtaining producing micro-organisms of methylated polyene macrolides which consists of interrupting the coding gene for the cytochrome P450 monooxygenase involved in the formation of the free carboxyl- group - gene homologous to the gene described in the invention *rimG* in other strains - in producing micro-organisms of polyene macrolides containing free carboxylgroups, using any conventional method for that purpose (transformation, conjugation, electroporation, infection, etc.).

Moreover, in another aspect, the invention relates to the use of any vector for carrying out a disruption of the coding gene for the cytochrome P450 monooxygenase involved in the formation of the free carboxyl- group in producing micro-organisms of polyene macrolides containing free carboxylgroups, belonging to, among others, by way of illustration and without limiting the scope of the invention, the following group: amphotericin B, nystatin, rimocidin, pimaricin and candicidin with the aim of producing the corresponding methylated polyenes. Among the micro-organisms suitable for use for the production of methylated polyenes as a result of the interruption of the coding gene for the cytochrome P450 monooxygenase involved in the oxidation of the methyl- group to carboxyl-, by way of illustration and without limiting the scope of the invention, we count on *Streptomyces nodosus,* a natural producer of amphotericin B, *Streptomyces rimosus,* a producer of rimocidin, *Streptomyces griseus,* a producer of candicidin; *Streptomyces natalensis,* a producer of pimaricin; *Streptomyces noursei,* a producer of nystatin.

Moreover, the invention relates to the suitable expression of genes which might have been affected by a polar effect when carrying out the disruption of the corresponding gene, using any vector as vehicle (replicative plasmids, integrative plasmids, actinophages, etc.) and being introduced by any of the conventional methods (transformation, conjugation, electroporation, infection, etc.). Illustrative, though non-limiting, examples of producing micro-organisms of polyene macrolides containing a free carboxyl- group include species of *Streptomyces*, for example *S. noursei, S. rimosus, S.* nodosus, *S. natalensis* and *S. griseus.*

The micro-organisms genetically handled as stated above, hereinafter the genetically handled homologous producing micro-organisms of methylated polyene macrolides of the invention, form part of the present invention and constitute an additional aspect thereof.

In another aspect, the invention relates to a method for producing a methylated polyene macrolide which comprises cultivating a genetically handled micro-organism of the invention under conditions which will permit the production of said methylated polyene and, if wished, to isolate and purify said compound which, by way of illustration and without limiting the scope of the invention, belongs to the following group: methylated amphotericin B, methylated nystatin, methylated pimaricin, and methylated candicidin. Another object of the invention consists of any of these novel methylated polyenes, methylated amphotericin B, methylated nystatin, methylated pimaricin, and methylated candicidin which can be used for the preparation of biocide and pharmacological compositions as in the case of rimocidin C and CE-108C.

Said genetically handled micro-organisms will be cultivated in any medium suitable for the fermentation of those micro-organisms and will generally contain one or more sources of carbon, one or more sources of nitrogen, one or more inorganic salts that can be assimilated by the micro-organism, and, if necessary, one or more nutrients such as vitamins and amino acids, dissolved in an aqueous medium, and the appropriate conditions will be applied (aeration/stirring, temperature and fermentation time, stages, etc.) for the growth of the micro-organisms and the production of the polyene macrolides. The polyene macrolides obtained are advantageously secreted into the culture medium from where they can be recovered by means of conventional techniques, for example, by means of using chromatographic methods, optionally prior to withdrawal of the cell extract. The polyene macrolide/s of interest can be separated on the basis of its/their physical-chemical characteristics, which permits a method to be designed for its/their purification starting from the culture medium following the fermentation process.

### Compound AB-400 (IVb)

The compound AB-400 (IVb), the amide corresponding to pimiracin (IVa), is a known natural product coming from *Streptomyces costae* (Cañedo L. M. et al. 2000, J., Antibiot. (Tokyo) 53: 623-626).

Trials conducted by the inventors with AB-400 (IVb) and pimiracin (IVa) have revealed that AB-400 displays a substantial increase in fungicide activity without this giving rise to haemolytic activity towards human red globules, which permits it to be stated that it displays greater selective toxicity towards membranes containing ergosterol than its non-amidated homologue pimiracin (IVa).

Other trials conducted by the inventors have revealed that amidated polyenes are significantly more soluble in water than their non-amidated homologues. This characteristic, together with the pharmacological properties described earlier, mean that this compound is suitable for clinical use, for topical or systemic treatment of mycosis or parasitosis, and also in the agro-alimentary industry.

Therefore, in another aspect, the invention relates to a pharmaceutical composition comprising the compound AB-400 (IVb) together with, optionally, one or more pharmaceutically acceptable excipients. If wished, said pharmaceutical composition can furthermore contain one or more therapeutic agents which might possibly boost the therapeutic action of said compound AB-400 (IVb) or increase its spectrum of action.

Said pharmaceutical composition can be used for preventing and/or treating infections caused by pathogenic organisms of humans or animals possessing cell membranes containing ergosterol, for example, parasites and pathogenic fungi of humans or animals.

In another specific embodiment, therefore, said pharmaceutical composition is an antiparasite composition and can be used in the prevention and/or treatment of infections caused by parasites whose cell membranes contain ergosterol, for example, *Trypsanoma, Leichmania,* etc. If wished, said antiparasite composition can furthermore contain one or more antiparasite agents which might possibly boost the therapeutic action of said compound AB-400 (IVb) or increase its spectrum of action.

In another specific embodiment, said pharmaceutical composition is an antifungal composition and can be used in the prevention and/or treatment of infections caused by fungi (whose cell membranes contain ergosterol). If wished, said antifungal composition can furthermore contain one or more antifungal agents which might possibly strength the therapeutic action of said compound AB-400 (IVb) or which increase its spectrum of action. Illustrative, though not limiting, examples of said antifungal agents include polyenes, such as amphotericin B, nystatin, AB-400 (IVb), allylamines (e.g., terbinafine, nafthafine, etc.), amorofia, tolnaftalate, etc., azoles such as chlotrimazol, miconazol, ketconazol, fluconazol, itraconazol, etc., benzofurans, for example griseofilvin, etc., pyrimidines, for example fluocytosin, etc.

The compound AB-400 (IVb) will be present in said pharmaceutical composition in a therapeutically effective quantity, in other words, a quantity suitable for exerting its therapeutic effect. In a particular embodiment, the pharmaceutical composition provided by this invention contains between 0.01% and 99.99% by weight of a compound of AB-400 (IVb) and can be presented in any suitable pharmaceutical form of administration depending on the chosen administration route, for example, oral, parenteral or topical. A review of the different pharmaceutical forms of administration of drugs and their preparation methods can be found in, for example, Tratado de Farmacia Galénica, C. Faulí i Trillo, 1st edition, 1993, Luzán 5, S. A. de Ediciones.

Therefore the invention also relates to the use of AB-400 (IVb) in the preparation of a medicine for the prevention and/or treatment of infection caused by pathogenic fungi of humans or animals whose cell membranes contain ergosterol, for example human or animal parasites or pathogenic fungi of humans or animals.

Likewise, in another aspect, the invention also provides a method for preventing and/or treating infections caused by pathogenic organisms of human or animals which possess a cell membrane containing ergosterol, for example, parasites and fungi that are pathogens of humans or animals, which comprises the stage of administering to an animal or human being in need of treatment a therapeutically effective quantity of a pharmaceutical composition provided by this invention which contains the compound AB-400 (IVb).

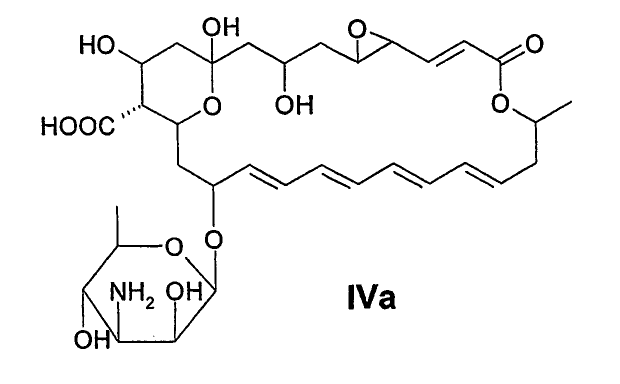

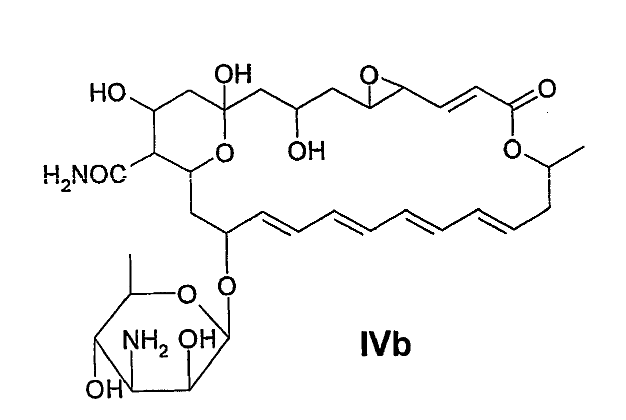

The following example illustrates the invention and must not be considered as limiting the scope thereof.

### EXAMPLES OF EMBODIMENT

### EXAMPLE 1.- Production and characterisation of rimocidin B (I-1a) and CE-108B (I-1b)

### 1. Experimental methods

### Bacterial strains and growth conditions

The bacterial strains and plasmids are shown in Table 1.

*Streptomyces diastaticus* var. 108 and its derivatives by genetic modification were grown in the routine way in liquid and solid medium SYM2 (Atlas R.M., Microbiological Media. CRC Press, Boca Raton, Florida) for the analysis of the production of tetraenes, and in liquid medium TSB (Oxoid) for the extraction of plasmids and total DNA.

*Streptomyces lividans* TK21 was used as general host for cloning and was grown in a solid medium R5 and in liquid medium YEME as described in field manuals (Kieser T et al., 2000, Practical Streptomyces Genetics, Norwich).

The strains of *E*. *coli* were grown in Luria-Bertani (LB) agar or in LB cultures as described in the specialised literature (Maniatis T. et al., 1982, Molecular Cloning, A Laboratory Manual, Cold Spring Harbor Press, Cold Spring Harbor N.Y.).

*P. chysogenum, C. krusei, A. niger., C. albicans* and *C. neoformans,* fungi used for testing antifungal activity, were grown in MPDA medium (composition: 2% malt extract, 2% glucose, 0.1% Bactopeptone).

**Table 1**

| **Bacterial strains and plasmids used in the invention** | | |
|---|---|---|
| **Strain or plasmid** | **Properties** | **Reference** |
| *Streptomyces diastaticus* var. 108 | Wild strain, producer of rimocidin and CE-108 | (a) |
| *S. diastaticus* var. 108/922 | Strain derived from wild *S. diastaticus* var. 108 by transformation with the plasmid plJ922 (control) | This invention |
| | | |
| *S. diastaticus* var. 108/PM1-500 | Strain derived from the wild with the gene *rimA* interrupted by integration of the phage PM1-500; non-producer of tetraenes | (b) |
| | | |
| *S. diastaticus* var. 108/743B | Strain derived from the wild by transformation with the plasmid pSM743B; producer of CE-108, rimicidin, CE-108B and rimocidin B | This invention |
| | | |
| *S. diastaticus* var.::PM1-500/743B | Strain derived from the wild with the gene *rimA* interrupted by integration of the phage PM1-500 and transformed with the plasmid pSM743B; producer of CE-108, rimicidin, CE-108B and rimocidin B | This invention |
| | | |
| *S. diastaticus* var. 108/784 | Strain derived from the wild by transformation with the plasmid pSM784; producer of CE-108, rimicidin. CE-108B and rimocidin B | This invention |
| | | |
| *S.* sp. RGU5.3 | Wild strain, producer of pimaricin and AB-400 | This invention |
| | | |
| *E. coli* JM101 | General cloning host | (c) |
| | | |
| *S. lividans* TK21 | General cloning host | (d) |
| | | |
| *Penicilium chrysogenum* ATCC10003 | Antifungal activity trials | ATCC |
| | | |
| *Candida albicans* ATCC10231 | Antifungal activity trials | ATCC |
| | | |
| *Candida krusei* ATCC 14243 | Antifungal activity trials | ATCC |
| | | |
| *Aspergillus niger* ATCC1004 | Antifungal activity trials | ATCC |
| *Cryptococcus neoformans* ATCC10226 | Antifungal activity trials | ATCC |
| | | |
| pIJ922 | Vector based on the replicon SCP2*, *tsr,* 24 kb | (c) |
| | | |
| pIJ941 | Vector based on the replicon SCP2*, *tsr, hyg,* 25 kb | (d) |
| | | |
| pNAe-1 | Gene *erm*E cloned in pUK21 | This invention |
| | | |
| pHis1 | Vector replicative in *E. coli,* transporting the promoter xylanase *xysA(xysAp),* Ap^{R}, 3.7 kb | (f) |
| | | |
| pSM736 | Fragments of 1.2 kb SacI-DraIII and 4.9 kb DraIII-BgIII which contain *rimA* simultaneously cloned in sites Sacl/ BamHl of pIJ2925 | This invention |
| | | |
| pSM738 | Fragment of 6.1 kb Bglll-Pstl of pSM736 (gene *rimA*) and fragment of 1.7 kb Pstl-Sphl of pNAe-1 (resistance gene *ermE*) simultaneously cloned in sites Smal/Sphi of pHisI | This invention |
| | | |
| pSM743B | Fragment of 8.4 kb BgIII of pSM738 (with contains *xysAp, rimA and ermE)* cloned in the site EcoRV of pIJ922 (see Figure 1) | This invention |
| | | |
| pGAe-1 | *ermE* gene closed in pSL1180 | This invention |
| | | |
| pSM784 | Fragment of 1.8 kb Sspl-EcoRV of pGAe-1 (resistance gene *ermE*) cloned in the site EcoRV of plJ941 (see Figure 1) | This invention |

| | | |
|---|---|---|
| Ap. amphicilin; Km. kanamycin; *ermE, tsr* and *hyg:* resistance genes to erythromycin, thiostrepton and hygromycin B (a) Perez-Zuñiga. F. J., et al., 2004, J. Antibiot. (Tokyo) 57: 197-204 (b) Seco E. M., et al., 2004, Chem. Biol. 11: 357-366 (c) Yanisch-Perron C. et al., 1985 Gene 33: 103-119 (d) Kieser T et al., 2000, Practical Streptomyces Genetics, Norwich (e) Lydiate D. J., et al., 1985 Gene 34: 223-235 (f) Ruiz-Arribas, A., 1997, Appl. Environ. Microbiol, 63: 2983:2988 | | |

### Genetic methods

The strains of *E. coli* were grown and transformed as described in Maniatis *et al.* (1982). The strains of *Streptomyces* were handled as described above (Kieser T., et al., 2000, cited earlier). The intraspecific conjugation was carried out by jointly growing the donor and receiver strains in a solid medium R5 without selection and later on selecting on the basis of the corresponding resistances to antibiotics of the plasmids and to the genetic markers. The handling of DNA was carried out as described earlier (Maniatis T, et al., 1982, cited earlier).

### Trials for the production of tetraenes

The production of tetraenes was analysed by extracting all of an aliquot of the culture with methanol as described previously (Seco E.M., et al., 2004, cited earlier). The extracts were filtered and subjected to HPLC analysis with a Waters 600S Controller equipment, fitted with Waters 996 PDA; the quantitative determination and the chromatographic conditions were the same as described previously (Pérez-Zúñiga F.J. et al., 2004, cited earlier).

### HPLC-MS trials

The mass spectra were determined with equipment 1100MSD HPLC connected to a quadrupole detector, the Agilent Technology Detector, using electrospray as source and a positive ionisation mode. The chromatographic conditions were the same as described previously (Pérez-Zúñiga F.J. et al., 2004, cited earlier).

### Purification of the novel compounds

*Streptomyces diastaticus* var. 108/784 or micro-organisms possibly modified with constructions capable of producing amidated polyenes (as has been stated earlier) was or were cultivated in either solid or liquid medium SYM2 (Pérez-Zúñiga F.J. et al., 2004, cited earlier). After six days, the complete solid medium in which the micro-organisms were cultivated was fragmented by shearing and made to pass through a 50 mL syringe; the solid medium thus fragmented was extracted with four volumes of methanol previously acidified with 25 mM formic acid. The cultures coming from the liquid medium were freeze-dried before carrying out similar extractions with methanol. The aqueous suspension was stirred for 1 hour and centrifuged at 5,000 g for 20 minutes in order to eliminate solid particles in suspension. The transparent supernatant was concentrated by means of roto-evaporation at 10-20 x 10⁶ units per microlitre (µL) measured at a wavelength of 304 nanometres (nm). The sample was then stored in 80% methanol/water until use. Two hundred millilitres of liquid cell culture, or a plate (24 x 24 cm) whose culture had previously been extracted with 5 volumes of methanol, gave rise to a production of up to 40 mg of the mixture of amidated tetraenes. The samples extracted in methanol were taken to 20% methanol with water and filtered in order to eliminate the precipitated material. The filtrate was slowly applied to an Omnifit column (250 x 25 mm, Supelco Cat No. 56010) previously packed with an ion exchange resin, such as SP-Sepharose, Phast Flow (Pharmacia) which was previously balanced with the same solution. Under these conditions the rimocidin and CE-108 were eluted with the front not attached to the column packing, as with the pigments coming from the culture; the corresponding amidated polyenes rimocidin B (I-1a) and CE-108B (I-1b) remained completely retained. The column, containing the compounds of interest retained in the solid phase, was exhaustively washed with the same solution in order to eliminate those compounds that did not interact with the SP Sepharose packing. The amidated polyenes rimocidin B (I-1a) and CE-108B (I-1b) retained by interaction with the column were eluted with 300 mM ammonium acetate pH 5 in 20% methanol with fractions being collected at regular times. Of the eluted fractions a selection was made of those which contained the mixtures of amides of interest; these were combined together and subjected to a physical process of desalination, using for this Sep-Pak cartridges (Waters). Finally, the desalinated fractions were dissolved in 20% methanol. The fraction containing the mixture of amidated polyenes (15 mg) which had been desalted was finally fractionated by HPLC (with the aim of separating the amidated polyenes); for this a semi-preparative column was used (Supelcosil PLC-8, 250 x 21.2 mm). The chromatographic parameters and the mobile phases, controlled with an automated gradient controller (Waters Automated Gradient Controller) were: 12 minutes with 100% of B (20 mM ammonium acetate pH 5, 20% ethanol), 43 minutes in a binary gradient of up to 50% of A (methanol) and 50% of B (curve 6 specified in the Waters chromatographic controller); 35 minutes in a binary gradient of up to 100% of A (curve 8, the same controllers as specified above), and a constant flow of 5 mL/min. The fractions were collected at regular intervals (5 mL per fraction) and those which contained the purified isolated compounds were subjected to an additional stage of desalination, as described above, and were finally freeze-dried twice. AB-400 was also purified (Cañedo L. M. et al. 2000, J., Antibiot. (Tokyo) 53: 623-626) starting from liquid cultures of *Streptomyces* sp. RGU5.3 as described above.

### Trials of haemolytic activity

The trials were conducted according to the method described by Gómez-Gómez et al. (Gómez-Gómez, J.M. et al., 1996, Mol. Microbiol. 19: 909-910). The samples of polyenes were first dried and then dissolved in DMSO at an estimated concentration of 10 to 30 mg/mL. Increasing quantities of the different polyenes were taken to a final volume of 100 µL of DMSO and mixed by means of gentle stirring with 500 µL of PBS buffer (Gómez-Gómez, J.M. et al., 1996, cited earlier), containing 2.5% of human blood, or eventually that of horse. After incubation at 37°C for 30 minutes without stirring, the cells were sedimented by centrifugation and the degree of haemolysis was assessed by means of measuring the absorption at 545 nm. The values corresponding to the total haemolysis were estimated with a suspension of 2.5% of horse blood in distilled water. The human blood (fundamentally erythrocytes) was obtained from local blood banks (Hospital Ramón y Cajal, Madrid); the horse blood came from Oxoid (defibrinated blood). The amphotericin B and the nystatin were obtained from Sigma (catalogue numbers A-4888 and N-3503, respectively) and the pimaricin came from Calbiochem (527962). All these polyenes were tested directly from commercial samples, without any additional purification

### II. Results

### Generation of the recombinant gene rimA

The gene *rimA* (Seco E.M. et al., 2004, Chem. Biol. 11: 357-366), coded within a polycistronic mRNA, was cloned under the control of the promoter *xysA* (*xys*Ap) of the gene of the xylanase of *Streptomyces halstedii* JM8 (Ruiz-Arribas, A., 1997, Appl. Environ. Microbiol, 63: 2983-2988). To do this, the promoter xysAp was rescued starting from pHis1 as a fragment *Bg*II/Smal of 547 pairs of bases which, moreover, carries the terminator of the methylenomycin resistance gene (T1: Adham S.A. et al, 2001, Arch. Microbiol. 177: 91-97) located in a position forward of the promoter *xys*Ap. After various stages described in Table 1, following the usual procedures in DNA cloning, the fragment of DNA which contained the gene *rimA* and the 3' end of the gene *riml* (positions 9336 to 15445 pairs of bases starting from the sequence deposited in the GeneBank under access number AY442225, as indicated in Figure 1; Seco, E.M. et al., 2004, cited earlier) was fused with xysAp in the proper orientation that permitted the *rimA* to be expressed under the promoter *xys*Ap (recombinant gene *rimA*). Finally, the gene *ermE* of pNAe-1 (Table 1) was rescued by digestion with the flanking enzymes and the resulting fragment of DNA was cloned in intermediate stages after the "recombinant *rimA*" gene in order to generate, in the final construction, a fragment of DNA containing (see Figure 1A) from left to right: the gene *rimA* under the control of the promoter xysAp, the fragment of truncated gene *rim*l and finally the complete gene *ermE.* The resulting fragment of DNA was cloned via blunt ends, according to the usual technique in Molecular Biology, in the unique restriction site *Eco*RV, located within the thiostrepton resistance gene of the *Streptomyces* vector pIJ922. The resulting plasmid (pSM743B, Figure 1A) confers resistance to erythromycin but not to thiostrepton due to having the corresponding thiostrepton resistance gene interrupted by insertion of the DNA fragment described above.

The correct functionality of the recombinant gene *rimA* cloned in pSM743B, as indicated above, was checked by introduction of the plasmid pSM743B in a mutant of *Streptomyces diastaticus* var. 108 previously generated by disruption of the native gene *rimA (Streptomyces diastaticus* var. 108/PM1-500, described in Seco, E.M. et al., 2004, cited earlier). The resulting genetic recombinant (generated by introduction of the plasmid pSM743B in a mutant of *Streptomyces diastaticus* var. 108 with the gene *rimA* previously interrupted) is capable of producing the native macrolide polyenes (rimocidin and CE-108) and the novel macrolide polyenes (rimocidin B and CE-108B). Likewise, the plasmid pSM743B introduced in the wild strain also induced the production of the four tetraenes (amidated and carboxylated) but with an increase in the total production of polyenes due probably to the expression of the biosynthetic genes of the cluster *rim.* Parallel with this, the plasmid pSM784 was constructed. To do this, the gene *ermE* was cloned, following the usual technology in Molecular Biology, and in successive stages, in suitable vectors of *Escherichia coli* for obtaining the complete gene *ermE* able to be rescued as a DNA fragment with blunt ends. Finally the DNA fragment containing the gene *erm*E in a DNA fragment with blunt ends was cloned in the unique restriction site *Eco*RV of the vector pIJ941. The resulting vector confers resistance to erythromycin and to hygromycin B though it is sensitive to thiostrepton due to having the corresponding resistance gene to it interrupted by the inactivating insertion of the fragment of the gene *ermE* (see Figure 1 B). When the plasmid pSM784 is introduced into the wild strain *Streptomyces diastaticus* var. 108, the resulting recombinant micro-organism is capable of producing the novel amidated polyenes rimocidin B (I-1a) and CE-108B (I-1b) as well as rimocidin B (IIa) and CE-108B (IIb).

Therefore, the modified genetics of *Streptomyces diastaticus* var. 108 containing the vector pSM743B or the vector pSM784 produce both the original polyenes (rimocidin and CE-108) and the new amidated polyenes (CE-108B and rimocidin B), with a similar chromatographic profile, analysed in HPLC, as indicated in Figure 1 C. In spite of the fact that qualitatively both genetic recombinants produce the same polyenes, the production of them is significantly greater in those recombinants containing the gene *rimA* and the erythromycin gene (plasmid pSM743B).

The production of both tetraenes (rimocidin and CE-108) was restored in the mutant *Streptomyces diastaticus* var. 108 with the gene *rimA* interrupted, when introducing the plasmid pSM743B, indicating that the recombinant gene *rimA* is functional. Nevertheless, the production of polyenes was significantly lower in this complemented mutant than in the wild carrier strain of the same plasmid pSM743B (Table 1); this lower production was not significantly altered when either glucose or xylane were added to the medium. These results seem to suggest that the expression of *rimA* would be a limiting stage in the production of the polyenes; this limiting stage is clearly overcome by the increase in the gene dose.

These results clearly indicate that the gene *erm*E in the vector derived from SCP2* plays a fundamental role for generating the novel polyenes. It has to be emphasised that neither the erythromycin resistance gene (*erm*E), cloned in other vectors such as pHJL401 (Larson J.L. et al., (1986) Plasmid 15. 199-209) nor a vector derived from SCP2* independently are sufficient for producing the novel structures.

### Characterisation of the novel polyenes

### HPLC-MS analysis

HPLC analysis was conducted combined with mass spectrometry of the fermentation culture of *S. diastaticus* var. 108/743B and *S. diastaticus* var. 108::PM1-500/743B; the masses deduced for the two novel tetraenes were 738 and 766 for the lowest and highest retention times, respectively. In both cases, the masses of the novel polyenes are a unit lower than that of the polyenes with the closest retention times, CE-108 (739) and rimocidin (767). Both the mass difference and the chromatographic mobility support the idea that the novel polyenes derive from natural macrolides.

### Elucidation of the chemical structure:

With the aim of elucidating the chemical structure, the novel polyenes were characterised on a preliminary basis with the aim of developing a method for their purification. Both compounds interacted not just with silica gel in the reverse phase as C8 and C18 but also with an ion exchange resin such as SP-Sepharose, and it turned out that these compounds had an accessible positive charge. This primary characterisation permitted a simple purification method to be designed starting from the fermentation culture of *Streptomyces diastaticus* var. 108/pSM743B or *Streptomyces diastaticus* var. 108/784, indifferently (see the section relating to Experimental Methods).

The compound CE-108B (I-1b) was obtained as a powder with a visible spectrum typical of tetraenes (λₘₐₓ = 317, 302, 289 nm), similar to that of CE-108 (IIb) (Pérez-Zúñiga F.J., et al. 2004, J. Antibiot. (Tokyo) 57: 197-204). The spectrum of ¹H-NMR with three signals in the *sp²* range at δ 6.25 (dd, 14.9, 10.9 Hz), a multiplet at δ 6.00-6.15 and a doublet doublet at δ 5.87 (15.2, 8.4 Hz) was similar to that of CE-108. Two exchangeable protons appeared at δ 7.30 and 6.83 as broad singlets. In the aliphatic range of δ 1.40-2.50, the spectrum shows a complex multiplet pattern, and the signals of three methyl- triplets and doublets, respectively, appeared at δ 1.17, 1.15 and 0.83. The mass spectrum (+)-ESI MS revealed the existence of pseudo-molecular ions at *m*/*z* 739 ([M+H]⁺) and 761 ([M+Na]⁺) which corresponds to the molecular formula C₃₇H₅8N₂O₁₃ by means of high resolution (found 739.40110, calculated 739.401715 for [M+H]⁺). The magnetic resonance spectrum ¹³C-NMR indicated 37 carbon signals as in CE-108, as required by the molecular formula. The data on ¹³C-NMR for CE-108 and CE-108B were closely related and permitted it to be concluded that CE-108 and CE-108B possessed the same carbon skeleton including the amino-sugar. According to these data, the second nitrogen has to be attributed an amide function, which identifies CE-108B (I-1b) as the amide of CE-108 (IIb).

The rimocidin B (I-1a) powder was dissolved in DMSO. The mass spectrum (+)-ESI MS determined the molecular weight of rimocidin B (I-1a) as being 766, which, by means of high resolution, corresponds to the molecular formula C₃₉H₆2N₂O₁₃ (found 767.43254, calculated 767.43301 for [M+N]⁺). The spectrum of ¹H-NMR was similar to that of CE-108B (I-1b) and showed two exchangeable protons H/D at δ 7.30 and 6.83, two doublet doublets and a multiplet in the interval δ 6.40-5.80. The aliphatic region was very complex due to the lower resolution, but a triplet and a doublet at δ 1.83 and 1.16 were easily identified, attributed to methyl- signals. The spectrum ¹³C-NMR indicated the presence of 39 carbon signals. The comparison with CE-108B (I-1b) revealed the presence of three carbonyl signals at 208.8, 174.1 and 172.1, in addition to *sp²* signals of 8 carbon atoms in the interval 136.7-128.3, and that of two acetal groups. The close similarity with CE-108B (I-1 b) finally identified this compound as the amide of rimocidin, identified in this description as rimocidin B (I-1a).

### Biological activities of the novel amidated polyene compounds

### Antifungal activity trials:

The antifungal activity of the novel amidated tetraenes [rimocidin B (I-1a) and CE-108B (I-1b)] was tested against various fungi: *Penicillium chrysogenum, Candida albicans, Aspergillus niger, Candida krusei* and *Cryptococcus neoformans.* Increasing quantities of the different tetraenes, dissolved in methanol, were applied to paper discs (9 mm in diameter); following the application, the discs were dried and placed on bioassay plates on which the corresponding test fungi had previously been spread. The activity of these amidated tetraenes was compared with that of the molecules from which they derived [rimocidin B (IIa) and CE-108B (IIb)], showing that the biological activity of the amidated polyenes was substantially greater than that of the corresponding tetraenes that they derived from (Figure 2) in all tested fungi. In all cases, the substitution of the free carboxyl- group for the amide- group increased the antifungal activity by approximately fourfold.

### Toxicity trials

In the above experiments it is clear that the modification of the novel amidated polyenes, produced by the genetic recombinants of *Streptomyces diastaticus* var. 108 gave rise to compounds with high antifungal activity. With the aim of determining whether the toxicity was also increased, determinations were carried out of the haemolytic activity of the novel amidated polyenes in comparison with the compounds produced by the wild strain. Human erythrocytes were used as cell models for this study (Cybulska B, et al., 2000, Acta Biochim. Pol. 47: 121-131; Gómez-Gómez, J.M. et al., 1996, Mol. Microbiol. 19: 909-910). The haemolytic activity of the novel compounds was assessed (see the section relating to Experimental Methods) against rimocidin and CE-108; amphotericin B and nystatin A were also included. As shown in Table 2, the haemolytic activity of the amidated tetraenes [rimocidin B (I-1a) and CE-108B (I-1b)] was not significantly different from that of the corresponding tetraenes that they derived from, while their antifungal activity was clearly greater. It is worth while highlighting the differences in toxicity observed between CE-108 (IIb) and CE-108B (I-1b) with rimocidin (IIa) and rimocidin B (I-1a); while 50% of haemolysis is achieved with 40 to 60 nanomoles for the last two polyenes, a concentration of CE-108 and is amide 6 or 7 times greater is required in order to achieve the same degree of haemolysis. Therefore, the pharmacological properties of the amidated polyenes CE-108B are significantly improved: while CE-108 displays low antifungal activity, its corresponding amidated derivative (CE-108B) has an antifungal activity increased to levels almost as high as that of rimocidin, while its haemolytic activity is 6 or 7 times lower. These trials were also carried out with horse blood and showed similar results (data not shown).

The same results were obtained comparing pimaricin (IVa) and its amidated derivative AB-400 (IVb). Both compounds were purified starting from the isolated strain *Streptomyces* sp. RGU5.3, as indicated in the section on Experimental Methods. The strain was cultivated in a medium supplemented with both glucose and sodium acetate, and it was found that the production of the amidated derivative was highly increased when the sodium acetate was added to the culture. In this way, using a fermentation culture containing glucose as the source of carbon and not sodium acetate, the pimaricin/AB-400 balance is 70/30; when the same medium is supplemented with sodium acetate, the production profile is reversed, being AB-400 (IVb) the main polyene compound produced (Figure 3). This effect was not observed for the production of amidated polyenes produced by the genetically modified strain *Streptomyces diastaticus* var. 108. The amidated polyene was purified starting from this medium and was tested for both antifungal and haemolytic activities. The results, summarised in Figure 3C, agree with the results observed previously with the amidated polyenes obtained from the genetic recombinants of *Streptomyces diastaticus* var. 108: while no antifungal activity was observed for pimaricin (IVa) at the tested concentration, the same quantity of AB-400 (IVb) was highly active; differences in activity were observed of close to 1 order of magnitude when *Penicillium chrysogenum* was used. Nevertheless, the trials of haemolytic activity conducted with AB-400 (IVb) and commercial pimaricin did not show significant differences between the two polyenes. Taking this data overall, it can be concluded that the amidated derivative of pimaricin offers pharmacological advantages over carboxylated pimaricin.

**Table 2**

| **Comparative haemolytic activity of various polyenes. The values tested for each polyene is given in nanomoles (left-hand column) and the corresponding haemolytic activities as a total percentage of haemolysis (see Experimental Methods)** | | | | | | |
|---|---|---|---|---|---|---|
| | **Amphotericin B** | **Nystatin A** | **Rimocidin (IIa)** | **Rimocidin B(I-1a)** | **CE-108 (IIb)** | **CE-108B (I-1b)** |
| **1** | 1.97 | | | | | |
| **2** | 4.47 | | | | | |
| **3** | 82.26 | | | | | |
| **4** | 100 | | | | | |
| **20** | | 2.20 | 10.00 | 10.00 | | |
| **40** | | 49.53 | 21.18 | 33.51 | | |
| **60** | | 76.80 | 65.02 | 84.80 | | |
| **80** | | 92.79 | 95.59 | 94.80 | | |
| **100** | | 100.00 | 100.00 | 100.00 | | |
| **120** | | | | | 11.72 | 12.01 |
| **160** | | | | | 14.48 | 15.86 |
| **200** | | | | | 16.60 | 23.29 |
| **240** | | | | | 26.89 | 30.22 |
| **280** | | | | | 32.47 | 34.86 |
| **320** | | | | | 44.25 | 40.39 |
| **360** | | | | | 63.56 | 61.34 |
| **400** | | | | | 82.12 | 88.26 |
| **440** | | | | | 100.00 | 100.00 |

### III. Discussion

The biosynthesis is described of two novel amidated polyenes with changes in the carboxyl- group produced by means of genetic manipulation of a natural produce organism of two mostly non-amidated polyenes: rimocidin and CE-108.

*Streptomyces diastaticus* var. 108, a producer of two natural tetraenes (rimocidin and CE-108) acquires the capacity to produce, naturally and as main compounds, the corresponding amides (rimocidin B and CE-108B) if it is suitably modified by means of genetic manipulation. As with other semi-synthetic polyene derivatives, the conversion of the free carboxyl- group into an amide- group entails a clear improvement in some of their pharmacological properties (substantial increase in antifungal activity but not in haemolytic activities), thus providing a significant advantage as antifungal agents in comparison with native tetraenes. This chemical modification in both derivatives entails an increase in the selective toxicity for the membranes of organisms whose composition includes ergosterol. the inventors have emphasised that a similar result is obtained with pimaricin (IVa) and AB-400 (IVb), which reinforces the idea that the substitution of the carboxyl- group in the polyenes for am amide- group will also increase the relative toxicity of other polyenes.

For the biosynthesis of these novel amidated polyenes, at least two possible mechanisms can be postulated: (a) the amides would be the result of an amidotransferase activity subsequent to the assembly of the polyketonic chain of the aglycone ("adornment" activity or post-PKS modification), which could be activated under the experimental conditions described in this invention, or (b) a malonamyl-CoA transferase activity would, by means of condensation module 7 of the corresponding PKS (Seco E.M., et al., 2004, Chem. Biol. 11: 357-366), incorporate malonamyl-CoA instead of methylmalonyl-CoA as proposed in the biosynthetic model for the production of CE-108 and rimocidin. In this latter case, a Claysen type non-decarboxylating condensation would be required, as occurs in biosynthetic thiolases (Heath & Rock, 2002, Nat. Prod. Rep. 19: 581-596), for the incorporation of malonamide in the polyketonic chain; in this case the *in vivo* availability of malonamide as condensing unit would be crucial for a good incorporation in the growing polyketonic chain. It is worth while highlighting that the producing strain also biosynthesises oxytetracycline, whose postulated "starter" unit for the polyketonic chain is malonamide. So in this strain, this metabolite would be easily available for the production of other secondary metabolites, as well as of oxytetracycline.

Although the genetic mechanism leading to the production of CE-108B and rimocidin B is not known, it seems clear that it at least requires the plasmids derived from SCP2* (such as pIJ922 or pIJ941) and the gene *ermE.* It has recently been described that sub-inhibitory concentrations of erythromycin can modulate the bacterial transcription (Goh et al., Proc. Nat.. Acad, Sci. USA 99: 17025-17030). Nevertheless, the possibility that the erythromycin can modulate the expression of a possible transcriptional regulator making the amidation stage possible in a producing organism of polyene can be discarded since the amides were also detected in cultures in which no erythromycin had been added. This opens up the possibility that the product of the gene *ermE* (a methylase) can act on another intermediate gene, coded within the DNA of the plasmids derived from SCP2* (pIJ922 or pIJ941), the end result of which would be the activation of a chromosome gene responsible for the amidation of the natural polyenes of *Streptomyces diastaticus* var. 108 (rimocidin and CE-108). The present invention provides a system for generating novel amidated polyenes by means of biotransformation by a genetically modified strain. This process, satisfactorily applied to the biosynthetic pathway of commercial polyenes, would undoubtedly be a simple process for the production of improved pharmaceutical compounds. Given the complexity of the polyene structures that this invention relates to, the biotransformation proposed in this invention for generating amidated polyene compounds constitutes a process that is undoubtedly more efficient than those described so far by means of organic synthesis for generating some semi-synthetic structures.

### EXAMPLE 2.- Production and characterisation of rimocidin C (IIIa) and CE-108C (IIIb)

### I. Experimental methods

### Bacterial strains, cloning vectors and growth conditions

The bacterial strains and the plasmids are shown in Table 1.

*Streptomyces diastaticus* var. 108 and its derivatives were cultivated in medium SYM2 (Atlas R.M., Microbiological Media. CRC Press, Boca Raton, Florida). *Streptomyces lividans* TK21 was used for the propagation of phages and as host strain, and was grown in solid medium R5 and in liquid medium YEME as described in field manuals (Kieser T et al., 2000, Practical Streptomyces Genetics, Norwich).

The strain *E. coli* JM101 was grown in Luria-Bertani (LB) agar or in LB culture as described in the specialised literature (Maniatis T. et al., 1982, Molecular Cloning, A Laboratory Manual, Cold Spring Harbor Press, Cold Spring Harbor N.Y.).

*Penicillium chysogenum* ATCC10003 was used for testing the antifungal activity and was grown in MPDA medium (composition: 2% malt extract, 2% glucose, 0.1% Bactopeptone).

**Table 1: Bacterial strains and plasmids used in the invention**

| **Strain, plasmid or phage** | **Properties** | **Reference** |
|---|---|---|
| | | |
| *E. coli* JM101 | General cloning host | a |
| | | |
| *Penicillium chysogenum* | Strain used in antifungal tests | ATCC1000 3 |
| | | |
| *S. lividans* TK21 | General cloning host in Streptomyces | b |
| | | |
| *S. diastaticus* var. 108 | Wild strain, producer of rimocidin and CE-108 | c |
| | | |
| *S. diastaticus* var. 108::PM1-768 | Derivatives of wild *S. diastaticus* var. 108 with the gene rimG interrupted by insertion of the phage PM1-768 | This invention |
| | | |
| *S. diastaticus* var. 108::PM1-768/743B | Previous strain where the plasmid pSM743B has been introduced; producer of rimocidin C and CE-108C | This invention |
| | | |
| *S. diastaticus* var. 108/743B | Derivative of wild *S. diastaticus* var. 108 in which where the plasmid pSM743B has been introduced | This invention (Example 1) |
| | | |
| *S. diastaticus* var. 108/PM1-702B | Derivative of wild *S. diastaticus* var. 108 with the gene *rimE* interrupted by integration of the phage PM1-702B | This invention |
| | | |
| *S. diastaticus* var. 108::PM1-702B/743B | Previous strain where the plasmid pSM743B has been introduced | This invention |
| | | |
| *S. diastaticus* var. 108/784 | Derivative of wild *S. diastaticus* var. 108 in which where the plasmid pSM784 has been introduced | This invention (Example 1) |
| | | |
| *S. diastaticus* var. 108/PM1-500 | Derivative of wild *S. diastaticus* var. 108 with the gene rimA interrupted by integration of the phage PM1-500 | d |
| | | |
| *S. diastaticus* var. 108::PM1-500/784 | Previous strain where the vector pSM784 has been introduced | This invention |
| | | |
| pHJL401 | Vector for cloning; it contains the replicon SCP2* and pUC19; it contains the resistance gene to thiostrepton. Bifunctional *E. coli*/*Streptomyces* | e |
| | | |
| pEL-1 | DNA fragment *Eco*R1*-Hind*III of pIJ4090 corresponding to the promoter *ermEₚ** (ref. b) cloned in the site *Eco*R1-*Hind*III of the vector pHJL401 | This invention |
| | | |
| PM1 | Actinophage derived from ϕC31 (att'), *hyg, tsr* | f |
| | | |
| pSM743B | Recombinant vector, derived from pIJ922, containing the gene *rimA* under the control of the xylanase promoter XysA | This invention (Example 1) |
| | | |
| pCNB5006 | Vector derived from pIJ2925, containing the promoter *ermEₚ** | d |
| | | |
| pSM721 | DNA fragment of 0.7 kb (*Sac*II) internal to the gene *rimG* cloned in the site *Hinc*II of pIJ2925 | This invention |
| | | |
| pSM768 | DNA fragment of 0.7 kb *Hind*II*-Xba*I of the vector pSM721 cloned in the site *Bam*HI-*Xba*I of pCNB5006 | This invention |
| | | |
| PM1-768 | DNA fragment *Bgl*II-*Pst*I of 1.0 kb of the vector pSM768 cloned in the site *Bam*Hl-*Pst*l of the phage PM1 | This invention |
| | | |
| pEL-1/702 | DNA fragment *Sal*l of 0.8 kb internal to the gene *rimE* cloned in the site *Bam*Hl of the vector pEL-1 | This invention |
| | | |
| PM1-702B | DNA fragment *Eco*Rl-*Xba*l of 1.1 kb of the vector pEL-1/702 cloned in the site *Ecl*136II of the phage PM1 | This invention |

| | | |
|---|---|---|
| a Yanisch-Perron et al., 1985 Gene 33: 103-119 b. Kieser et al., 2000, Practical Streptomyces Genetics, Norwich c. Pérez-Zúñiga, et al., 2004, J. Antibiot. (Tokyo) 57: 197-204 d. Seco et al., 2004, Chem. Biol. 11: 357-366 e. Laron et al., 1986, Plasmad. 15: 199209 f. Malpartida et al., 1986, Mol. Gen. Genet. 205: 66-77 | | |

### Genetic methods

The *E. coli* strain JM101 was grown and transformed using the protocols described above (Maniatis et al., 1982, Molecular Cloning, A Laboratory Manual, Cold Spring Harbor Press, Cold Spring Harbor N.Y.). The strains of *Streptomyces* were handled as described above (Kieser T. et al., 2000, cited earlier). The intraspecific conjugation was carried out as has been described above in Example 1. The handling of DNA was carried out as described earlier by Maniatis et al., 1982 (cited earlier).

### Trials for the production of tetraenes

The production of tetraenes was analysed as described in Example 1. The HPLC analysis was carried out under the same conditions as described previously (Pérez-Zúñiga F.J. et al., 2004, cited earlier).

### HPLC-MS trials

The mass spectra were determined with equipment 1100MSD HPLC connected to a quadrupole Agilent Technology Detector, using electrospray as source and a positive ionisation mode. The chromatographic conditions were the same as described previously (Pérez-Zúñiga F.J. et al., 2004, cited earlier).

### NMR analysis

The NMR spectra were measured in a Varian Inova 600 spectrometer (5999.740 MHz). The ESI mass spectra were recorded on Finnigan LCQ equipment with a Rheos 4000 quaternary pump (Flux Instrument). The HR ESI mass spectra were measured with a Bruker FTICR 4.7 T spectrometer.

### Purification of the novel compounds

*Streptomyces diastaticus* var. 108::PM1-768/743B was cultivated in solid medium SYM2 (Pérez-Zúñiga F.J. et al., 2004, cited earlier), supplemented with thiostrepton (50 µg/ml) and erythromycin (25 µg/ml) for the correct selection of the insertion of the phase PMI-768 in the chromosome and presence of the plasmid pSM743B, respectively. After 6 days, the complete solid medium in which the micro-organisms were cultivated was fragmented as described previously in Example 1. CE-108C (IIIb) and rimocidin C (IIIa) were purified by HPLC using a semi-preparative column (Supelcosil PLC-8, 250 x 21.2 mm); the gradient applied was similar to that described previously for analytical fractionating (cited earlier) and controlled by an automated gradient controller (Waters Automated Gradient). The fractions containing the polyenes were combined together, subjected to a desalination stage using Sep-Pak cartridges (Waters), and were finally liofilized twice. This method can be used for purifying any methylated polyene starting from the culture of the corresponding genetically modified micro-organism.

### Trials of haemolytic activity

The trials were conducted on human blood enriched in eritrocytes, as described previously in Example 1.

### Preparation of cell-free extracts and "in vitro" amidation trials

For the preparation of cell-free extracts, the genetic recombinant *Streptomyces diastaticus* var. 108/784 and *Streptomyces* sp. RGU5.3, producers of amidated polyenes, were grown in 50 millilitres of medium SYM2 (Atlas *et al.,* Microbiological Media. CRC Press, Boca Raton, Florida) for three days. The mycelium was collected by centrifugation for 10 minutes at 5,000 g, 4°C; it was washed with a solution of 20% glycerol and resuspended in 15 ml of a 20% solution of glycerol. Aliquots of 500 microlitres were stored at -20°C until use. At the time of use, the cells were collected by centrifugation as described above and were resuspended in a buffer of 50 mM Tris-HCl, pH 7.5, 0.5 mM EDTA, 5% glycerol, 50 mM NaCl, 0.5 mM PMSF and 1 mM of betamercaptoethanol (TEPM buffer). The resuspended cells were subjected to disruption by ultrasound; the homogenate was clarified by centrifugation twice at 8,000 g for 15 minutes at 4°C. The polyenes contaminating the cell-free homogenate were partially eliminated by passing the supernatant through a Sep-Pak cartridge C18 (Waters). These fractions were subjected to fractionating with ammonium sulphate, taking them sequentially to 45% and 60% saturation. The precipitate from 60% saturation was dissolved at a concentration of four times with respect to the original and was used for the valuations of amidotransferase. The amidotransferase tests were performed on 200 microlitres containing 100 microlitres of the solution described above, 4 x 10⁶ Units of optical density measured at 340 nm of the solutions containing the different polyenes, 2.5 mM of glutamine, 25 mM of NaCl, 10 mM of MgCl₂, 4 mM of ATP and 125 mM of TES buffer at pH 7.2. The reactions were incubated at 30°C for 60 minutes and halted by the addition of 1 volume of methanol; the precipitate was eliminated by centrifugation at 4,000 g and analysed by HPLC as described in Pérez-Zúñiga, *et al.,* 2004, cited earlier.

### II. Results

### a) NOVEL METHYLATED POLYENES

### a.1.- Determination of the biosynthesis mechanism of polyene amides

Two possible alternative mechanisms of biosynthesis of rimocidin B (I-1 a) and CE-108B (I-1b) have been suggested earlier: (**a**) condensation of malonamyl-CoA elongation units in module 7 instead of the methylmalonyl-CoA units proposed for the formation of rimocidin (IIa) and CE-108 (IIb), and (**b**) an "adornment" activity (such as amidotransferase) which give rise to the amide starting from the lateral carboxyl- of rimocidin (IIa) and/or CE-108 (IIb). In order to decide between one and the other mechanism, it was considered that the role of gene *rimG* could be crucial: the disruption of the gene *rimG* would lead to a mutant capable of producing the corresponding amides due to being the carrier of a plasmid involved in the induction of the formation of the amidated tetraenes if the mechanism were (a) or incapable of this in the case of possibility (b).

So, a mutant was generated by disruption in the gene *rimG* using the actinophage PM1 as vector (Malpartida *et al.,* 1986, cited earlier). In order to prevent possible polar effects on genes located downstream of *rimG* (see Figure 4), a fragment of 0.6 kpb *Sac*II internal to *rimG* (coordinates 8267-8849 of the sequence deposited in GeneBank AY442225) was cloned after the fragment containing the promoter of the gene *erm*Eₚ, and the assembly in the phage PM1. The recombinant actinophage PM1-768B was used for infecting spores of *S. diastaticus* var. 108 giving rise to lysogenes *S. diastaticus* var. 108::PM1-768. These lysogenes did not produce any polyene, which suggested a lesser expression of the promoter *erm*Eₚ in comparison with the native promoter. Attempts to interrupt the gene *rimG* using a more powerful promoter such as *erm*Eₚ*^{*}* (Kieser *et al.,* 2000, cited earlier) have not been satisfactory. There are two genes located below the insertion point: *rim*H and *rimA.* Given that *rim*H codes a ferrodoxin whose role it has been proposed is to mediate the electron transport required by RimG (Seco *et al.,* 2004, cited earlier), it is reasonable to think that an appropriate expression of the gene *rim*A would probably be the critical parameter for restoring the production of polyenes. For this reason, the plasmid pSM743B, which is capable of complementing the disruption of *rim*A and inducing the formation of amidated polyenes (cited earlier) was transferred by intraspecific conjugation from the wild strain *S. diastaticus* var. 108/743B to the lysogene *S. diastaticus* var. 108::PM1-768, with *S. diastaticus* var. 108::PM1-768/743B being the resulting recombinant. Once the corresponding genotypes were confirmed, the fermentation cultures were analysed for the production of polyenes. There were two compounds that were mostly detected in the fermentation cultures; HPLC and mass spectra analysis determined that they were 709 and 737 mass units (30 units less than those of CE-108 (IIb) and rimocidin (IIa), respectively). The data suggested that they were tetraenes derived from CE-108 (IIb) and rimocidin (IIa) where the side carboxyl- of the macrolactone ring had been substituted for a methyl- group as a consequence of the disruption of *rimG.* The compounds have been called rimocidinC (IIIIa) and CE-108C (IIIb). The absence of amidated polyenes CE-108B (I-1b) and rimocidin B (I-1a) under these conditions where their production is induced clearly suggests that the formation of amidated derivatives previously requires the formation of the side carboxyl- group and that the formation of the amide- group is due to an "adornment" activity in place of condensation of malonamyl-CoA units in module 7 of the polyketide synthase. This "adornment" activity is probably due to an amidotransferase.

### a.2.- Elucidation of the chemical structures of rimocidin C (IIIa) and CE-108C (IIIb)

With the aim of elucidating the novel tetraenes rimocidin C (IIIa) and CE-108C (IIIb), they were both purified from the fermentation culture of *S. diastaticus* var. 108::PM1-768/743B using a reverse phase silica C8 column (see Experimental Methods).

The compound rimocidin C (IIIa) was obtained as a yellow powder which revealed the existence of pseudo-molecular ions at m/z 738 ([M+H]) and 760 ([M+Na]⁺) which corresponds to the molecular formula C₃₉H₆₃NO₁₂ by means of high resolution (found 738.442217, calculated 738.442300 for [M+H]). Compared with rimocidin (IIa) (C₃₉H₆₁NO₁₄), this compound corresponds to the loss of two atoms of oxygen and the addition of two protons in rimocidin C (IIIa), which can formally be interpreted as a substitution of the carboxyl- group in the carbon C-14 for a methyl- residue.

The spectrum of ¹H-NMR was very similar to that of rimocidin (IIa) and showed three signals in the range *sp²*, a doublet doublet (dd) at δ 6.30, a multiplet at δ 6.05-6.18, and a second dd at δ 5.90 (Table 2). The sugar protons appeared in the range δ 3.25-4.62. The aliphatic region of the spectrum ¹H-NMR also exhibited similarities with that of rimocidin (IIa), especially with respect to five complex multiplet patterns in the range δ 1.30-2.50. Four methyl-groups instead of three as in rimocidin (IIa) appeared as two triplets and two doublets at δ 0.90, 0.95 and 1.26, 1.00, respectively. The greatest difference between rimocidin (IIa) and rimocidin C (IIIa) was the presence of the methyl-doublet δ 1.00, which shows a signed H, H cross in the COSY spectrum with 14-H at δ 1.22 (δ_{C} 43.8).

The spectrum ¹³C-NMR indicated 39 carbon signals as in rimocidin (IIa), as required by the molecular formula. This and the similarities of the proton spectra permitted it to be concluded that rimocidin (IIa) and rimocidin C (IIIa) possess the same carbon skeleton including the sugar mycosamine, the only difference being the presence of two carbonyl groups instead of three in rimocidin (IIa). The signals δ 211.6 and 174.5 were attributed to a ketone- group and a lactone carbonyl, respectively. The coupling HMBC ³*J* of the proton at δ 5.03 (C-27) to the carbonyl at 174.5 confirmed the lactone, so rimocidin C (IIIa) has to be 14-decarboxy-14-methyl- rimocidin (I-1a).

The yellow powder of CE-108C (IIIb) was easily soluble in methanol. In this case either, the spectrum of ¹H-NMR exhibited similarities with those of rimocidin C (IIIa) and CE-108 (IIb). The aliphatic region exhibited the signals of four methyl- groups along with three doublets at δ 1.25, 1.23 and 0.99, and a triplet at 0,90, instead of three methyl- signals as in CE-108 (IIb). The mass spectrum (+)-ESI NS determined the molecular weight of CE-108C (IIIb) as being m/z 709, which corresponds to the molecular formula C₃₇H₅₉NO₁₂ by means of high resolution (found 710.410905, calculated 710.411000 for [M+H]⁺). The magnetic resonance spectrum ¹³C-NMR confirmed the presence of 37 carbon signals as in CE-108 (IIb), as required by the molecular formula. The comparison with the magnetic resonance spectrum ¹³C-NMR for CE-108B (I-1b) revealed the presence of just two carbonyl signals at δ 211.3 and 174.3, again attributed to a ketone- group and to a lactone carbonyl. The greatest difference in this case was the absence of carbonyl acid, which appeared at δ 179.3 in CE-108C (IIIb), and the presence of an additional methyl- signal at 13.7 belonging to the methyl- doublet at δ 0.99 in the spectrum of ¹H-NMR. A careful comparison of the data on CE-108 (IIb) and CE-108C (IIIb) clearly indicated that in this tetraene too, the carboxyl- group of C-14 of CE-108 (IIb) was replaced by a methyl- group, which identifies CE-108C (IIIb) as the derivative 14-decarboxy-methyl-CE-108.

### Biological activities of the novel amidated polyene compounds

The antifungal activity of the novel non-carboxylated tetraenes rimocidin C (IIIa) and CE-108C (IIIb) was measured against *Penicillium chrysogenum, Candida albicans, Aspergillus niger, Candida krusei* and *Cryptococcus neoformans,* in comparison with that of the native tetraenes rimocidin (IIa) and CE-108 (IIb). The antifungal activities of the two intermediaries, rimocidin C (IIIa) and CE-108C (IIIb), measured as stated in example 1, were not significantly different from those of the end products rimocidin (IIa) and CE-108 (IIb).

In order to measure whether other pharmacological properties of the novel tetraenes were different, haemolytic activity trials were conducted and compared with the parent compounds. Human eritrocytes, were used for these tests. The results are shown in Table 4. It can be pointed out that although the antifungal activities of rimocidin (IIa) and rimocidin C (IIIa) were similar, their toxicities (measured in terms of haemolytic activity) were 2.5-5 times lower for rimocidin C (IIIa) than for rimocidin (IIa). Increasing quantities were used for the haemolytic trials until reaching 600 nmoles of CE-108C (IIIb); the haemolysis detected was less than 20%, suggesting a lower toxicity of the non-carboxylated tetraene CE-108C (IIIb) than for any of the other tetraenes. This suggests an interesting improvement in the pharmacological properties.

**Table 2.- Comparative haemolytic activity of rimocidin (IIa), rimocidin C (IIIa), CE-108 (IIb) and CE-108C (IIIb). The quantities applied for each tetraene are stated in nanomoles (left-hand column). The values of the corresponding haemolytic activities are stated as percentages with respect to total haemolysis.**

| nm | Rimocidin (IIa) | Rimocidin C (IIIa) | CE-108 (IIb) |
|---|---|---|---|
| 20 | 15.55 | | |
| 40 | 44.57 | | |
| 60 | 73.82 | | |
| 80 | 85.01 | | |
| 100 | 100 | 8.27 | |
| 120 | | 21.07 | |
| 140 | | 53.96 | 7.83 |
| 160 | | 78.92 | 17.03 |
| 200 | | 84.7 | 22.64 |
| 240 | | 100 | 24.29 |
| 280 | | | 37.95 |
| 320 | | | 57.8 |
| 360 | | | 67.72 |
| 400 | | | 86.12 |
| 440 | | | 100 |

### b) DETERMINATION OF THE SUBSTRATE OF THE AMIDOTRANSFERASE ACTIVITY

### b.1.- Disruption of the gene rimE

With the aim of determining what was the substrate of the "adornment" activity or presumed amidotransferase, the gene *rim*E*,* which codes for the glycolsyltransferase responsible for the incorporation of the sugar mycosime into the macrolactone ring, was proceeded to be disrupted.

Owing to the fact that the gene *rim*E is transcribed in a polycistron of approximately 9 kb which also houses the genes *rimF, rimG, rimH* and *rimA* (Seco. E.M. et al., 2004, Chem. Biol. 11: 357-366) (Figure 4A), it was necessary to prevent a polar effect on genes located downstream in the chromosome. In order to effect the disruption, the powerful promoter *erm*Ep^{*} (Kieser *et al.,* 2000, cited earlier) was cloned in front of the fragment of 0.8 kpb *Sal*l internal to the gene *rim*E (coordinates 5629-6484 of the sequence deposited in GeneBank AY442225) in the correct orientation for permitting the transcription of the rest of the messenger RNA. The resulting construction was cloned in the phage PM1 in various steps described in Table 1. The resulting recombinant phase (PM1-702B) was used for infecting spores of *Streptomyces diastaticus* var. 108, permitting the isolation of lysogenes *S*. *diastaticus* var. 108::PM1-702B. The correct integration into the chromosome was confirmed by means of the Southern blotting technique. The analysis of fermentation cultures by HPLC and mass spectra analysis determined the presence of four majority compounds (see Figure 5) which correspond to the aglycones of CE-108 and rimocidin, and also the aglycones of rimocidin C (IIIa) and CE-108C (IIIb). When the plasmid pSM743B, which is capable of inducing the formation of the amides rimocidin B (I-1a) and CE-108B (I-1b), into the wild *S. diastaticus* var. 108, was introduced into this lysogene by conjugation, no formation of amidated aglycones was observed at all. The data is interpreted as if the substrate of the possible amidotansferase activity would really be rimocidin (IIa) andCE-108 (IIb) but not their aglycones.

### 2.- "in vitro" amidation trial of rimocidin (IIa) and CE-108 (IIb)

On account of all the data set out above, the conversion of rimocidin (IIa) and CE-108 (IIb) into their corresponding amides seems to be carried out by an amidotransferase "adornment" activity which uses as substrates both tetraenes rimocidin (IIa) and CE-108 (IIb) fully formed. In order to determine the presence of this activity, amidotransferase activity tests were conducted, using as substrates CE-108 (IIb) and rimocidin (IIa) purified by HPLC. In order to carry out the tests, the strain *S. diastaticus* var. 108/784, cited in Example 1, was grown in liquid medium SYM2 for 3 days, as stated in Experimental Methods, in order to obtain cell-free extracts. Different parameters were initially tested: substrates, enzyme co-factors, donors of the amide- group, optimum pH of the reaction. The reaction products were analysed by HPLC and the valuation was optimised using for the enzyme tests the sediment of various fractionations with ammonium sulphate, carried out according to the standard conditions of use in biochemical works in the field. Finally, the most optimum conditions found for the valuation of the amidotransferase activity are stated in the section on Experimental Methods. A clear conversion was observed both of CE-108 (IIb) and of rimocidin (IIa) into their corresponding amides rimocidin B (I-1a) and CE-108B (I-1b), respectively (see Figure 6A and 6B). The identity of the compounds observed was determined by mass spectrometry analysis. The data permitted it to be determined that the "adornment" activity is actually an ATP dependent amidotransferase activity which uses glutamine as donor of the amide- group. The activity could be detected not just in the extracts of *S. diastaticus* var. 108/784 [producer of rimocidin (IIa), rimocidin B (I-1a), CE-108 (IIb) and CE-108B (I-1b)] but also in extracts of *Streptomyces* sp. RGU5.3 [producer of pimaricin (IVa) and AB-400 (IVb)], whose amidotransferase activity turned out to be similar to that of *S. diastaticus* var. 108/784.

In order to determine the substrate specificity in the amidotransferase activity, the cell-free extracts of *S. diastaticus* var. 108/784 were also tested against heterologous substrates such as amphotericin B and pimaricin (IVa) though the results were only satisfactory using pimaricin (IVa) as substrate. As shown in Figure 6C, the cell-free extracts of *S. diastaticus* var. 108/784 were capable of converting pimaricin (IVa) into its corresponding amide AB-400 (IVb). Nevertheless, it was not possible to detect amidotransferase activity of cell-free extracts of *Streptomyces* sp. RGU5.3 using rimocidin (IIa), CE-108 (IIb) or amphotericin B as substrates, and the only compound capable of amidating was pimaricin (IVa) (Figure 7). The data permits the conclusion to be drawn that the amidotransferase activity of *S. diastaticus* var. 108/784 has a wider range of substrate recognition than *Streptomyces* sp. RGU5.3.

### DEPOSIT OF BIOLOGICAL MATERIAL

A culture of the micro-organism *Streptomyces diastaticus* var. 108/784, corresponding to the wild strain *S. diastaticus* var. 108 to which the plasmid pSM784 has been introduced, was deposited in Deutsche Sammlung von Mikroorganismen und Zellkulturen GmbH (DSMZ), Braunschweig, Germany, on 14 March 2005. Its access number is DSM 17187. The strain contains the complete pathway for producing rimocidin (IIa) and CE-108 (IIb), and is furthermore capable of producing the corresponding amidates rimocidin B (I-1a) and CE-108B (I-1b).

A culture of the micro-organism *Streptomyces diastaticus* var. 108::PM1-768/743B was deposited in Deutsche Sammlung von Mikroorganismen und Zellkulturen GmbH (DSMZ), Braunschweig, Germany, on 18 July 2005. Its access number is DSM 17482. This strain is the producer of the methylated polyenes rimocidin C (IIIa) and CE-108C (IIIb).

The techniques habitually used for the genetic handling of the genus *Streptomyces* and the deposit of genes/promoters for their public access can permit the generation of the constructions described in this invention or other functionally equivalent ones. The vectors used are for public use and are routinely used in laboratories in which one habitually works with *Streptomyces.*

The sequence of the coding DNA for the genes that is mentioned in this invention is found deposited and publicly accessible in the GeneBank databases under access number AY442225.

## Claims

1. A polyene macrolide compound **characterised by** the formula (I): 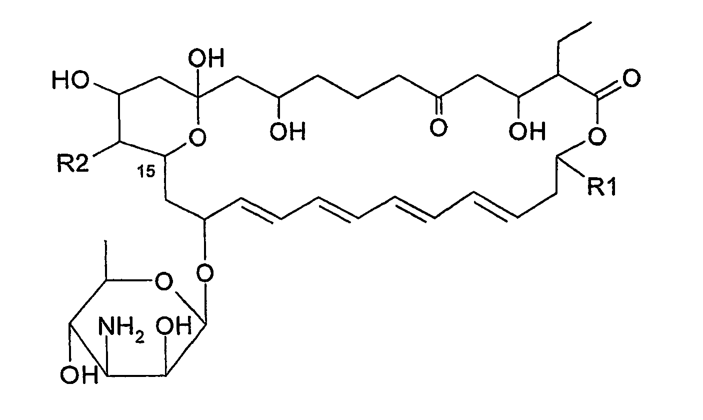 in which:
R1 is alkyl C₁-C₃;
R2 is a functional group chosen between CH₃- or CONH₂- (methyl- or primary amide-), its isomers, salts, prodrugs or solvates.

2. A compound according to claim 1 **characterised by** the formula (I-1) 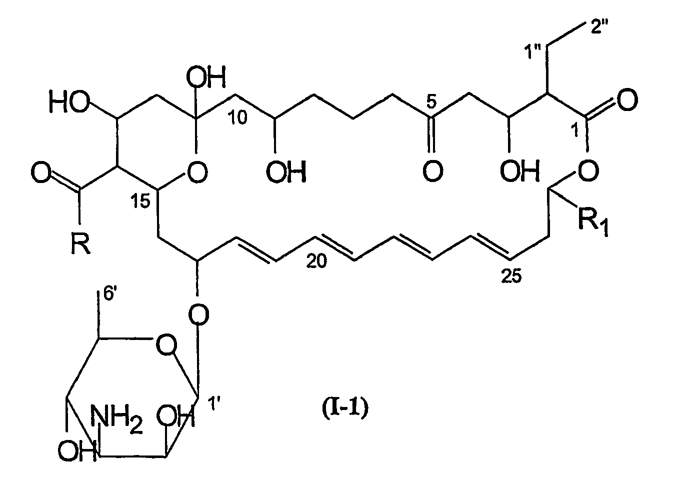 in which
R is NH₂; and
R1 is alkyl C₁-C₃
its isomers, salts, prodrugs or solvates.

3. Compound according to claim 2, **characterised in that** it is selected from among amidated compounds belonging to formula I identified as rimocidin B (I-1a) and CE-108B (I-1b):

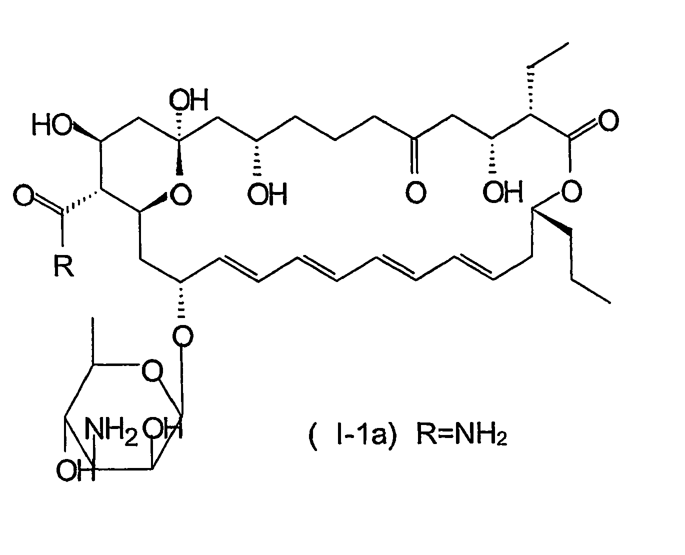

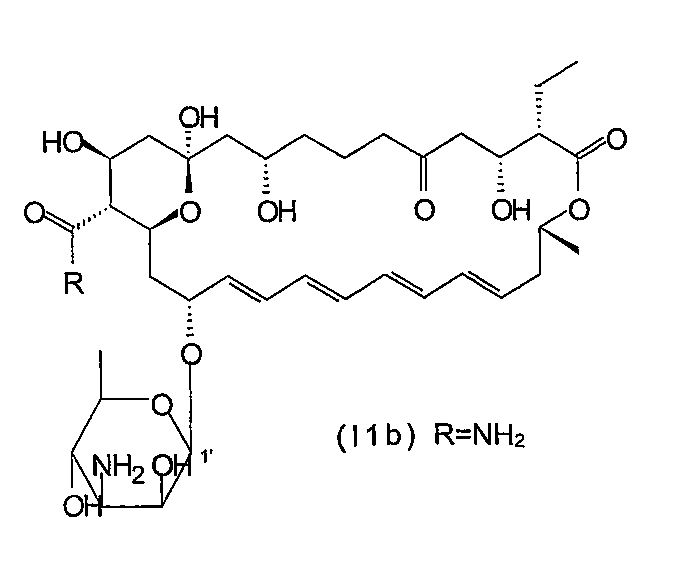

4. A compound according to claim 1, **characterised by** the formula (III): 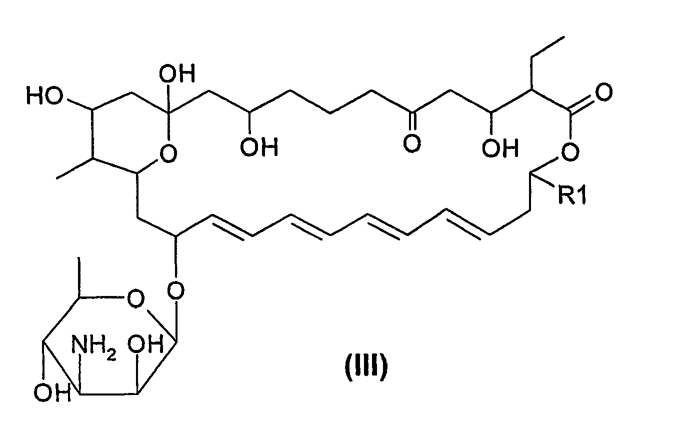 in which:
R₁ is alkyl C₁-C₃, its isomers, salts, prodrugs or solvates.

5. Compound according to claim 4, **characterised in that** it is selected from among the compounds belonging to formula III identified as rimocidin C (IIIa) and CE-108C (IIIb):

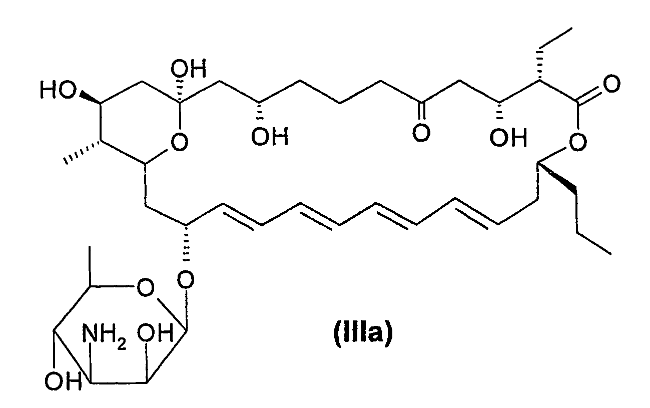

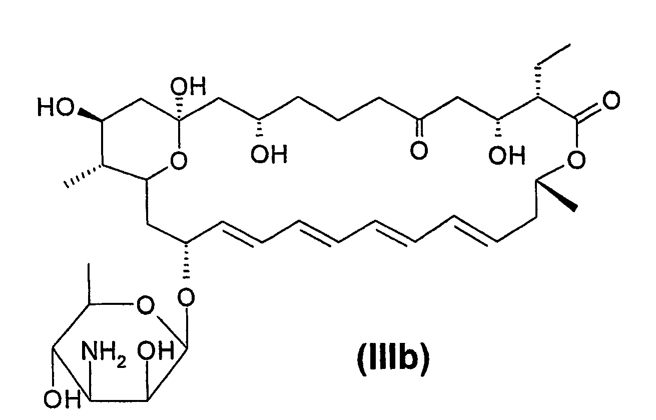

6. Biocide composition **characterised in that** it comprises a compound of formula (I) according to claim 1, together with an inert vehicle.

7. Biocide composition according to claim 6, **characterised in that** the compound of formula (I) is selected from among a compound of formula (I-1) according to claim 2.

8. Biocide composition according to claim 7, **characterised in that** the compound of formula (I-1) is selected from among rimocidin B (I-1a), CE-108B (I-1b) and their mixtures.

9. Biocide composition according to claim 6, **characterised in that** the compound of formula (I) is selected from among a compound of formula (III) according to claim 4.

10. Biocide composition according to claim 9, **characterised in that** the compound of formula (III) is selected from among rimocidin C (IIIa), CE-108C (IIIb) and their mixtures.

11. Pharmaceutical composition **characterised in that** it comprises a compound of formula (I) according to claim 1, along with, optionally, one or more pharmaceutically acceptable excipients.

12. Pharmaceutical composition according to claim 11, **characterised in that** the compound of formula (I) is selected from among a compound of formula (I-1) according to claim 2.

13. Pharmaceutical composition according to claim 12, **characterised in that** the compound of formula (I-1) is selected from among rimocidin B (I-1a), CE-108B (I-1b) and their mixtures.

14. Pharmaceutical composition according to claim 11, **characterised in that** the compound of formula (I) is selected from among a compound of formula (III) according to claim 2.

15. Pharmaceutical composition according to claim 12, **characterised in that** the compound of formula (III) is selected from among rimocidin C (IIIa), CE-108C (IIIb) and their mixtures.

16. Pharmaceutical composition according to any of claims 11 to 15, which furthermore comprises one or more therapeutic agents.

17. Use of a compound of formula (I) according to claim 1, in the preparation of a medicine for the prevention and/or treatment of human or animal infection caused by pathogenic organisms whose cell membranes contain ergosterol or which, without containing it, are sensitive to polyene macrolides.

18. Use according to claim 17, **characterised in that** the compound of formula (I) is selected from among a compound of formula (I-1) according to claim 2.

19. Use according to claim 18, **characterised in that** the compound of formula (I-1) is selected from among rimocidin B (I-1a), CE-108B (I-1b) and their mixtures.

20. Use according to claim 17, **characterised in that** the compound of formula (I) is selected from among a compound of formula (III) according to claim 4.

21. Use according to claim 20, **characterised in that** the compound of formula (III) is selected from among rimocidin C (IIIa), CE-108C (IIIb) and their mixtures.

22. Antifungal composition **characterised in that** it comprises a compound of formula (I) according to claim 1, together with, optionally, one or more inert vehicles.

23. Antifungal composition according to claim 22, **characterised in that** the compound of formula (I) is selected from among a compound of formula (I-1) according to claim 2.

24. Antifungal composition according to claim 23, **characterised in that** the compound of formula (I-1) is selected from among rimocidin B (I-1a), CE-108B (I-1 b) and their mixtures.

25. Antifungal composition according to claim 22, **characterised in that** the compound of formula (I) is selected from among a compound of formula (III) according to claim 4.

26. Antifungal composition according to claim 25, **characterised in that** the compound of formula (III) is selected from among rimocidin C (IIIa), CE-108C (IIIb) and their mixtures.

27. Composition according to any of claims 22 to 26, which furthermore comprises one or more antifungal agents.

28. A method for controlling infection caused by phytopathogenic fungi in a plant which comprises applying to said plant, or to the medium surrounding it, an antifungal composition according to any of claims 22 to 27.

29. A method for controlling infection caused by phytopathogenic fungi in a fruit which comprises applying to said fruit an antifungal composition according to any of claims 22 to 27.

30. A method for controlling infection caused by a fungus capable of developing in prepared food which comprises applying to said prepared food an antifungal composition according to any of claims 22 to 27.

31. A method for the production of a compound of formula (I-1) according to claim 2, **characterised in that** it comprises cultivating a micro-organism selected from among *Streptomyces diastaticus* var. 108/743B, *Streptomyces diastaticus* var. 108/784, *Streptomyces diastaticus* var. 108::PM1-500/743B and combinations of them, under conditions that permit the production of said compound of formula (I-1) and, if wished, to isolate and purify this said compound.

32. Method according to claim 31, **characterised in that** the compound of formula (I-1) to be produced is selected from between rimocidin B (I-1a), CE-108B (I-1b) and their mixtures.

33. Method according to claims 31 and 32, **characterised in that** jointly with the polyene macrolide of formula (I-1), preferably rimocidin B (I-1a), CE-108B (I-1b) or their mixtures, the starting polyene rimocidin (IIa), CE-108 (IIb) or their mixtures is simultaneously produced.

34. Method for the production of a compound of formula (III) according to claim 4, **characterised in that** it comprises the following stages:
- culture of the micro-organism *Streptomyces diastaticus* var. 108::PM1-768/743B under conditions that permit the production of compounds of formula (III)
- obtaining the fermentation culture and, if wished,
- the isolation and purification of those compounds formula (III).

35. Method according to claim 34, **characterised in that** the compound of formula (III) belongs to the following group: rimocidin C (IIIa), CE-108C (IIIb) and their mixtures.

36. Recombinant micro-organism necessary for carrying out the method according to claims 31 to 33, **characterised in that** it is selected from among *Streptomyces diastaticus* var. 108/743B, *Streptomyces diastaticus* var. 108/784 (DSM 17187) and *Streptomyces diastaticus* var. 108::PM1-500/743B.

37. Recombinant micro-organism necessary for carrying out the method according to claims 34 and 35, **characterised in that** it produces the compound of formula (III) according to claim 4 and **in that** it exclusively affects the expression of the gene rimG or a homologous gene.

38. Micro-organism according to claim 37, **characterised in that** it is the micro-organism *Streptomyces diastaticus* var. 108::PM1-768/743B.(deposit number: DSM 17482) and is the producer of the methylated polyenes of general formula (III) rimocidin C, CE-108C and their mixtures.

39. Micro-organism according to claim 38, **characterised in that** they are functional equivalent micro-organisms of the micro-organism *Streptomyces diastaticus* var. 108::PM1-768/743B (deposit number: DSM 17482).

40. A culture of a micro-organism **characterised in that** it is selected from among *Streptomyces diastaticus* var. 108/743B, *Streptomyces diastaticus* var. 108/784, *Streptomyces diastaticus* var. 108::PM1-500/743B, *Streptomyces diastaticus* var. 108:::PM1-768B/743B and combinations of them.

41. Use of a micro-organism according to claims 36 to 39, or of a culture of a micro-organism according to claim 40, in order to obtain a compound of formula (I) according to claim 1.

42. Use of a micro-organism according to claim 41, **characterised in that** the compound of formula (I-1) and (III) is selected from among rimocidin B (I-1a) or CE-108B (I-1b), between rimocidin C (IIIa) or CE-108C (IIIb), respectively, or among mixtures of those compounds.

43. The fermentation culture of a micro-organism according to claims 36 to 39 **characterised in that** it comprises a compound of formula (I).

44. Fermentation culture according to claim 43, **characterised in that** the micro-organism belongs to the following group: *Streptomyces diastaticus* var. 108/743B, *Streptomyces diastaticus* var. 108/784, *Streptomyces diastaticus* var. 108::PM1-500/743B and combinations of them and **in that** it comprises a compound of formula (I-1) according to claim 2 belonging to the following group: rimocidin B (I-1a), CE-108B (I-1b) and their mixtures.

45. Fermentation culture according to claim 43, **characterised in that** the micro-organism is *Streptomyces diastaticus* var. 108:::PM1-768B/743B and **in that** it comprises a compound of formula (III) according to claim 4 belonging to the following group: rimocidin C (IIIa), CE-108C (IIIb) and their mixtures.

46. A method for obtaining a recombinant producing micro-organism of polyene macrolides containing an amide- group according to claim 36 **characterised in that** it comprises introducing an expression vector producing micro-organisms of polyene macrolides containing free carboxyl- groups, or introducing a combinations of vectors containing, on the one hand,
(i) a vector which comprises a biosynthetic cluster of a polyene or a fragment of it, and, on the other hand,
(ii) a vector derived from SCP2* which comprises the gene *ermE* or a vector which comprises a replication origin different from that of SCP2*, the gene *ermE,* and a fragment of the vector SCP2*, in producing micro-organisms of polyene macrolides which have free carboxyl- groups.

47. A method for obtaining a micro-organism according to claims 37 to 39, **characterised in that** the resulting strain is exclusively affected in the expression of the gene *rimG* and **in that** it comprises the following stages:
a) obtaining of a mutant in the gene *rimG* of the micro-organism *S. diastaticus* var. 108 or of its equivalents by means of the disruption or deletion of said gene, incapable of producing polyenes, and
b) its later transformation with a vector, preferably a plasmid, capable of complementing the disruption of the gene *rimA* in the chromosome in said mutant.

48. Method for obtaining a micro-organism according to claim 46, **characterised in that** the resulting strain is the strain *S. diastaticus* var. 108::PM1-768/743B.

49. Method for obtaining a micro-organism according to claim 37, **characterised in that** the resulting strain is exclusively affected in the expression of the homologous gene *rimG* and **in that** it comprises the following stages:
a) obtaining of a mutant in the homologous gene of the original micro-organism by means of the disruption or deletion of said gene, incapable of producing polyenes, and
b) its later transformation with a vector, preferably a plasmid, capable of complementing the disruption of that gene in the chromosome in said mutant.

50. Method for obtaining a micro-organism according to claim 49, **characterised in that** the homologous gene of the gene *rimG* is a gene with cytochrome P450 monooxygenase activity belonging to the following genes *pimG, amphN, nysN, canC*).

51. Method for obtaining a micro-organism according to claims 46 and 50, **characterised in that** the original micro-organism is an Actinomycete.

52. Method for obtaining a micro-organism according to claim 51, **characterised in that** the Actinomycete is *Streptomyces* sp.

53. Method for obtaining a micro-organism according to claim 52, **characterised in that** the *Streptomyces* sp. belongs to the following group: *Streptomyces noursei, Streptomyces albidus, Streptomyces rimosus, Streptomyces diastaticus* var. 108, *Streptomyces nodosus, Streptomyces natalensis, Streptomyces chattanoogensis* and *Streptomyces. griseus.*

54. A recombinant micro-organism obtainable according to the method of any of claims 46 to 53.

55. An expression vector according to claim 46, **characterised in that** it is selected from among:
a) a vector derived from the vector SCP2*, or a fragment of it, which contains the replication origin of SCP2* and the erythromycin resistance gene *ermE*);
b) a vector which contains (i) the replication origin of the vector SCP2*; (ii) the gene *ermE,* and (iii) a fragment of the vector SCP2*;
c) a vector which contains (i) a replication origin, (ii) the gene *ermE,* and (iii) a fragment of the vector SCP2*, in which said replication origin is different from the replication origin of SCP2*;
d) a vector which lacks a replication origin and contains the gene *ermE,* and a fragment of the vector SCP2*;
e) a vector derived from the vector SCP2*, which contains (i) a replication origin, (ii) the gene *ermE;* and (iii) the entire biosynthetic cluster of a polyene or a fragment of said cluster;
f) a vector derived from the vector SCP2*, which contains (i) a replication origin, equal to or different from the replication vector SCP2 (ii) the gene *ermE;* (iii) a fragment of the vector SCP2*; and (iv) the entire biosynthetic cluster of a polyene or a fragment of said cluster;
g) a vector which lacks a replication origin and contains the gene *ermE,* and the entire biosynthetic cluster of a polyene or a fragment of said cluster; and
h) a vector which lacks a replication origin and contains (i) the gene *ermE;* (ii) a fragment of the vector SCP2*; and (iii) the entire biosynthetic cluster of a polyene or a fragment of said cluster.

56. Expression vector according to claim 55, **characterised in that** it is derived from a vector SCP2* and carrier of the erythromycin resistance gene *(ermE).*

57. Expression vector according to claim 56, **characterised in that** it is the plasmid pSM784.

58. Expression vector according to claim 55, **characterised in that** it comprises the replication origin of SCP2*, the erythromycin resistance gene (*ermE*) and a fragment of the vector SCP2*.

59. Expression vector according to claim 54, **characterised in that** it comprises a replication origin different from the replication origin of SCP2*, the erythromycin resistance gene *(ermE)* and a fragment of the vector SCP2*.

60. Expression vector according to claim 55, **characterised in that** it is a derivative of SCP2*, which comprises the gene *ermE* and the entire biosynthetic cluster of a polyene or a fragment of said cluster, under the control of a promoter.

61. Expression vector according to any of claims 57 to 59, **characterised in that** it furthermore comprises the entire cluster *rim* or a fragment of said cluster.

62. Expression vector according to claim 61, **characterised in that** it is the plasmid pSM743B.

63. Enzymatic method for obtaining an amidated polyene from polyenes with free carboxylated groups in the macrolactone ring **characterised in that** cell-free extracts of producing strains of amidated polyenes are used and **in that** it comprises the following stages:
a) adjustment of a mixture with a substrate consisting of a polyene with free carboxylated groups or mixtures of several of them, purified or not, and a protein extract coming from a producing strain or strains of amidated polyenes,
b) reaction of the mixture of a) under conditions of presence of ATP/Mg⁺⁺ and glutamine or else donors of amide- groups, and
c) purification of amidated polyenes.

64. Enzymatic method according to claim 63, **characterised in that** the amidated polyene to obtain is CE-108B (I-1b), rimocidin B (I-1a) or their mixtures, the substrate polyene of a) is CE-108 (IIb), rimocidin (IIa) or their mixtures, purified or not, and in which the extract of a) is obtained from the following strains: *S. diastaticus* var. 108/784 and *S. diastaticus* var. 108/743B.

65. Enzymatic method according to claim 63, **characterised in that** the amidated polyene to obtain is AB-400 (IVb), the substrate polyene of a) is pimaricin (IVa), purified or not, and in which the extract of a) is obtained from the following strains: *S. diastaticus* var. 108/784 and *S. diastaticus* var. 108/743B.

66. Enzymatic procedure according to claim 63, **characterised in that** the amidated polyene to obtain is AB-400 (IVb), the substrate polyene of a) is pimaricin (IVa), purified or not, and in which the extract of a) is obtained from the strain *Streptomyces* sp. RGU5.3.

67. Cell-free extract necessary for commencing the enzymatic method according to claims 63 to 67, for producers of amidated polyenes **characterised in that** it entails an amidotransferase activity capable of converting carboxylated polyenes into their corresponding amides *"in vitro"* and **in that** they come from producing strains of amidated polyenes.

68. Cell-free extract according to claim 67, **characterised in that** the following micro-organisms are obtained: *S. diastaticus* var. 108/743B, *S. diastaticus* var. 108/784 (DSM 17187) and/or *Streptomyces* sp. RGU5.3

69. Methylated polyene compounds **characterised in that** they belong to the following group: methylated amphotericin B, methylated nystatin, methylated pimaricin and methylated candicidin.

70. Use of the methylated polyenes according to claim 69 for the preparation of biocide and pharmacological compositions.

71. A method for producing a polyene macrolide **characterised in that** it comprises cultivating a recombinant micro-organism according to claim 54, under conditions that permit the production of said compound, and, if wished, to isolate and purify said compound.

72. Method according to claim 71, **characterised in that** the polyene macrolide is selected from among a polyene macrolide containing a free carboxyl- group, a polyene macrolide containing a free amide- group and their mixtures.

73. Method according to claim 72, **characterised in that** the polyene macrolide which contains a free carboxyl- group is selected from among amphotericin B, nystatin, rimocidin, pimaricin, candicidin and their mixtures.

74. Method according to claim 72, **characterised in that** the polyene macrolide which contains an amide- group is selected from among the compound AB-400 (IVb) and a compound of formula (I) according to claim 1.

75. Method according to claim 74, **characterised in that** the compound of formula (I) is selected from among rimocidin B (I-1a), CE-108B (I-1b) and their mixtures.

76. Method according to claim 72, **characterised in that** the polyene macrolide is selected from among pimaricin (IVa), AB-400 (IVb), rimocidin (IIa), rimocidin B (I-1a), CE-108 (IIb), CE-108B (I-1b) and their mixtures.

77. A pharmaceutical composition **characterised in that** it comprises the compound AB-400 (IVb), along with, optionally, one or more pharmaceutically acceptable excipients.

78. Use of the compound AB-400 (IVb) in the preparation of a medicine for the prevention and/or treatment of human or animal infection caused by pathogenic organisms whose cell membranes contain ergosterol.

79. Use according to claim 78, **characterised in that** the human or animal pathogenic organisms whose cell membranes contain ergosterol are selected from between parasites and fungi.

80. Use of the medicine according to claims 78 and 79 for the treatment of human or animal systemic infection caused by pathogenic organisms whose cell membranes contain ergosterol.
